# EUROPEAN PATENT APPLICATION

(11) **EP 1 970 086 A2**
(43) Date of publication of application: **17.09.2008**
(21) Application number: 08250911.8
(22) Date of filing: 17.03.2008
(51) Int. Cl.: A61M 5/32, A61M 5/50

(54) **Injector for use with pre-filled syringes and method of assembly**

(30) Priority: 15.03.2007 US 724804
(71) Applicant: Bristol-Myers Squibb Company, Princeton NJ 08543-4000 (US)
(72) Inventor: David-Hegerich, Faith C., Holliston, MA 01746 (US); Bertram, Paul, Franklin, MA 02038 (US); Rollins, Nathan, Bolyston, MA 01505 (US); Uschold, Robert C., Leominster, MA 01453 (US); Kane, William J., Sutton, MA 01590 (US); Doucette, Thomas, Stow, MA 01775 (US); Johnson, Timothy N., Exeter, NH 03883 (US); Denzer, Michael, Ringoes, NJ 08551 (US)
(74) Representative: Butler, Michael John

(57) **Abstract**

An injection device (8) for use with a pre-filled syringe (26). The device features a track (42) and track follower (82) engagement which facilitates locking a protective needle guard (18) over the tip of the needle (30) at the conclusion of the injection. The device further includes a tamper evidence overcap (20) which, once removed from the device cannot be readily reinstalled. The injection device features a tubular handle (10) which is grasped by the hand and moved towards the injection site to administer the injection. The device is suitable for self-administration of injections.

## Description

### BACKGROUND OF THE INVENTION

### A. Field of the Invention

This invention relates generally to injection devices and more particularly to an injection device for use with a pre-filled syringe. The invention also relates to methods of assembly of an injection device and pre-filled syringe.

### B. Description of Related Art

A variety of devices for facilitating an injection of medicament into a human or animal subject are known in the art. Most of such devices provide a mechanism by which the needle tip is covered by a guard after completion of the injection in order to prevent an accidental needle stick. The following patent references are considered representative of the art: Larsen et al., U.S. patent 6,547,764; Restelli et al., U.S. patent publication 2005/0096595; Ranford, U.S. patent 4,994,045; Nussbaum, U.S. patent 5,088,986; Spencer, U.S. patent 4,801,295; Asbaghi, U.S. patents 6,884,237, 6,379,336, and 5,688,241; Walker et al., U.S. patent 4,932,940; Vaillancourt, U.S. patent 5,591,138; Doyle, U.S. patent 6,623,459, and U.S. patent publication 2005/0119624; Perez et al., U.S. patents 6,344,032 and 6,159,184; Firth et al., U.S. patent 5,616,134, and Mahurkar, U.S. patent 5,338,311.

The art has also developed so-called "pen injectors", also known as auto-inject pens, self-injectors, and auto-injectors, which are designed to facilitate self-administration of injections. Examples of pen injectors are described in Giambattista et al., U.S. patent application publication 2005/0277895 and Karlsson, U.S. patent application publication 2005/261634. In pen injectors, the device is typically designed such that the needle is penetrated into the skin manually by the user and subsequently the user presses a button, usually on the top of the injector, in order to inject the medicament. Alternatively, the needle is penetrated automatically and the medicament is injected when the button is pressed. Abbott Laboratories has recently developed a pen injector device for injecting the arthritis drug Humira ®. A summary description of the device can be found at http://www.humira.com/pen/f/.

Other art of interest includes Stroup, U.S. patent 6,742,246, which is directed to a system for assembling injection devices.

### SUMMARY OF THE INVENTION

The following embodiments and aspects thereof are described and illustrated in conjunction with systems, tools and methods which are meant to be exemplary and illustrative, not limiting in scope.

In a first aspect, an injection device is provided for use in administering an injection to a human or animal subject. The device includes a syringe assembly containing a medicament to be administered to the subject and having a needle extending from the syringe assembly, the needle having a tip. The device further includes a needle guard surrounding the syringe and moveable relative to the syringe. The needle guard has at least one track follower. The device further includes a hollow tubular handle having an interior region receiving the needle guard and syringe. A plunger is coupled to and positioned within the interior region of the handle. The plunger could be a separate piece or integrally formed in the handle. The plunger and handle are moveable relative to the syringe body during administration of an injection. The plunger has a first end engaging a stopper in the syringe assembly to expel medicament from the syringe upon movement of the plunger and handle relative to the syringe.

The device further includes at least one track for engagement with the track follower of the needle guard. The track may be formed on the plunger or on the handle. A spring is provided which acts on the syringe and the needle guard. The track follower moves along the track during movement of the handle and plunger relative to the needle guard during the injection. The track further includes a lock position (or lock pocket). The spring urges the needle guard relative to the handle and plunger at the completion of the injection such that the track follower moves into the lock position to lock the needle guard relative to the handle and plunger, with the needle guard covering the tip of the needle to prevent an accidental needle stick following completion of the injection.

In another aspect, an injection assembly is provided for use with an injection device having a track follower. The injection assembly includes a syringe body containing a medicament to be administered to the subject and having a needle extending from the syringe body. The assembly also includes a plunger. The plunger has a first end which engages a stopper in the syringe to expel medicament from the syringe upon movement of the plunger relative to the syringe. The plunger further includes at least one track for engagement with a track follower of the injection device. The track has a lock position to lock the injection device in a condition such the injection device covers the needle at the completion of an injection. In one possible embodiment the injection assembly (plunger and pre-filled syringe) is assembled with a previously manufactured injection device to form a completed injection device which is ready to ship to end users or distributors.

As will be explained below, the location of the track on the plunger and the track follower in the injection device (e.g., on the needle guard) can be interchanged. Thus, in another aspect, an injection assembly is provided for use with an injection device. The assembly includes a syringe comprising a body containing a medicament to be administered to the subject and having a needle extending from the syringe body, and a plunger. The plunger further includes at least one track follower for engagement with a track incorporated into the injection device. The track has a lock position to lock the injection device in a condition such the injection device covers the needle at the completion of an injection.

In yet another aspect, an injection device is provided for receiving a syringe and plunger combination. The syringe contains a medicament for administration to a human or animal subject. The syringe includes a needle and a needle sheath (also referred to as a needle shield). The syringe is coupled to a plunger when the injection device is ready for use. The injection device includes a hollow tubular handle having an open first end and a needle guard positioned within the handle. The needle guard has at least one track follower extending from the needle guard and engagement features (e.g., snaps) for engagement with the syringe. The assembly further includes a removable tamper-evidence overcap assembly. The overcap assembly further includes a means for engaging the needle guard and preventing re-installation of the overcap onto the needle guard once the overcap has been removed from the needle guard. The injection device further includes a feature or device within the overcap assembly (e.g., in the form of a retainer or other device with gripping fingers, snaps or other features) which engages the needle sheath when the syringe and plunger combination are inserted into the injection device assembly. The needle sheath is removed from the needle simultaneous with the action of removing the overcap assembly from the needle guard.

In yet another aspect, an improvement to an injection device for injection of a medicament from a syringe into a human or animal subject is provided. The improvement comprises at least one track and at least one track follower. The track includes a feature, e.g., rib, wall or other feature, wherein the track follower contacts the feature when the medicament is substantially completely expelled from the syringe during the injection. The track follower is at least partially resilient. The construction of the track, the feature and the track follower is such that the contact with the track follower with the feature produces an audible sound indicating to the user of the device that the injection of the medicament from the syringe is substantially completed.

In still another aspect, an apparatus for injection of a medicament from a syringe having a needle with a tip into a human or animal subject is provided. The apparatus includes an injection device supporting the syringe and facilitating injection of the medicament from the syringe. The injection device includes a needle guard, at least one track and at least one track follower. A spring is provided which acts on the syringe and the injection device. The track follower moves along the track during an injection using the device. The track further includes a lock position. The spring urges the needle guard such that the track follower moves into the lock position to lock the needle guard in an extended position at the completion of the injection, with the needle guard covering the tip of the needle to prevent an accidental needle stick following completion of the injection.

In yet another aspect of the invention, a method of assembly of an injection device is disclosed which comprising the steps of: 1) inserting a hollow tubular needle guard into a hollow tubular handle; 2) placing a tamper-evidence overcap over the needle guard; 3) placing a compression spring over a pre-filled syringe assembly; 4) inserting the compression spring and pre-filled syringe assembly combination into the needle guard and locking the spring and pre-filled syringe assembly to the needle guard; 5) inserting a plunger having a first end into the handle such that the first end of the plunger engages with stopper within the syringe assembly; and 6) locking the plunger to the handle. In one variation of this method, the syringe assembly includes a needle sheath covering a tip of a needle, and wherein step 2) comprises the sub-steps of: 2a) placing an inner tamper evidence overcap over the needle guard; 2b) placing a needle sheath-engaging device onto the inner tamper evidence overcap and needle guard combination; and 2c) placing an outer tamper evidence overcap over the inner tamper evidence overcap and needle shield-engaging device and securing the outer and inner tamper evidence overcaps to each other.

A further aspect of the invention relates to an improvement to an injection device having a syringe having a needle sheath, and a needle guard. The improvement comprises providing a tamper-resistant overcap covering the syringe and needle guard prior to use of the injection device, wherein the overcap is removed from the needle guard by applying an axial force to the overcap, and wherein the overcap is constructed with a feature which (1) pulls the needle sheath off the needle simultaneous with the removal of the overcap from the needle guard and (2) thereafter securely retains the needle sheath within the overcap.

Yet another aspect of the invention relates to an improvement to an injection device comprising a syringe having a tubular body for containing a medicament, a stopper moveable within the tubular body, a needle, a needle sheath covering the needle, and a plunger acting on the stopper to expel medicament from the syringe. The improvement comprises providing a feature in the injection device limiting the axial movement of the syringe relative to the plunger when the needle sheath is removed from the needle.

In addition to the exemplary aspects and embodiments described above, further aspects and embodiments will become apparent by reference to the drawings and by study of the following detailed descriptions.

### BRIEF DESCRIPTION OF THE DRAWINGS

Preferred embodiments of the invention are illustrated in the drawings. It is intended that the embodiments and figures disclosed herein are to be considered illustrative rather than restrictive.
Figure 1 is a side view of an injection device according to an embodiment of the invention, showing a tamper evidence overcap installed on a handle which is grasped by the user to make an injection. A pre-filled syringe, needle guard and other components of the device are located within the handle and/or covered by the overcap.
Figure 2 is a cross-sectional view of the injection device of Figure 1.
Figure 3 is another cross-sectional view of the injection device of Figure 1, taken in a plane orthogonal to the plane of Figure 2.
Figure 4 is an exploded view of the injection device of Figure 1.
Figures 5A-5D are several views of the plunger of Figures 2-4, and in particular Figure 5A is an end view of the plunger, Figures 5B and 5C are side views and Figure 5D is a perspective view.
Figure 6 is an exploded view of a pre-filled syringe which is inserted into the injector device of Figure 1 to form an assembly for giving an injection, including stopper, syringe body, needle, and needle sheath.

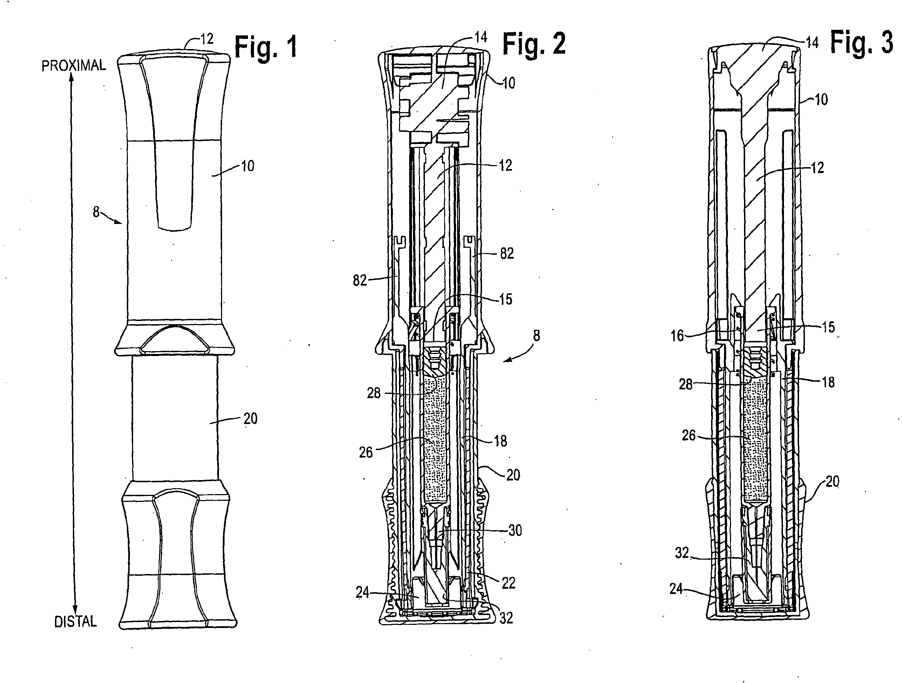

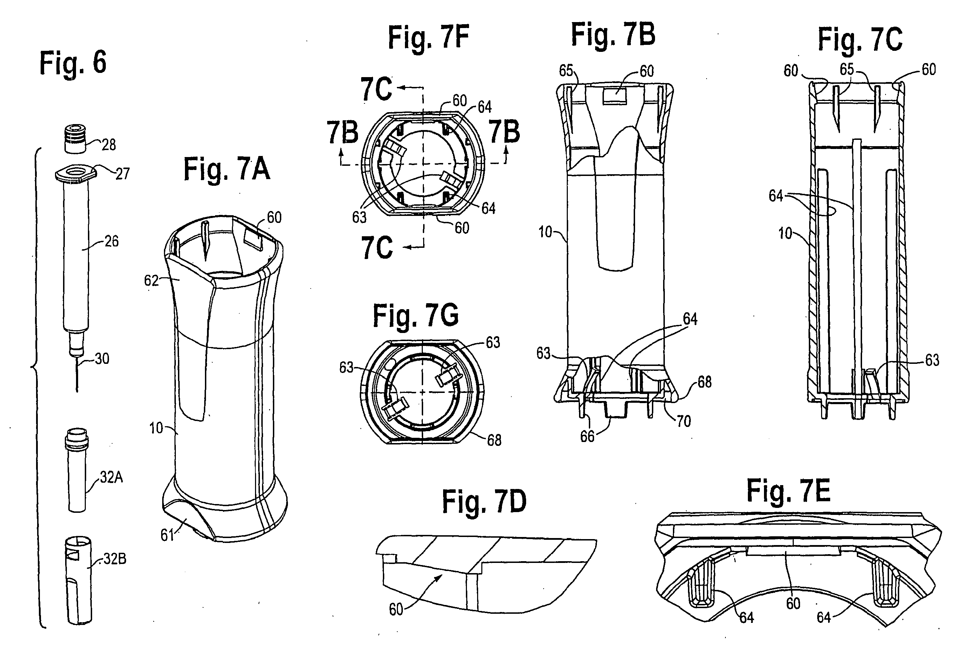

Figure 7A-7G are several views of the handle of Figures 1-4, and in particular Figure 7A is a perspective view, Figure 7B is a side view partially in cross-section along the lines 7B-7B of Figure 7F, Figure 7C is a cross-sectional view along the lines 7C-7C of Figure 7F, Figure 7D is a detail of the snap feature at the open end of the handle which retains the plunger to the handle at the time of assembly; Figure 7E is a detail of the snap feature of Figure 7D and also showing the rails which guide insertion of the needle guard during assembly; Figure 7F is an end view of the handle as seen from the top of the handle, and Figure 7G is an opposite end view.

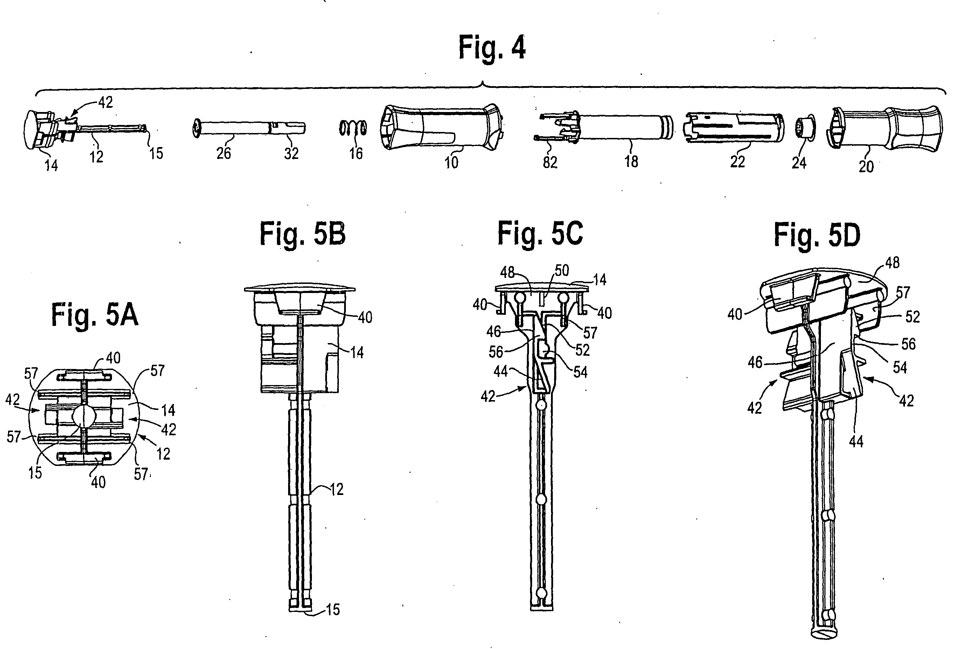

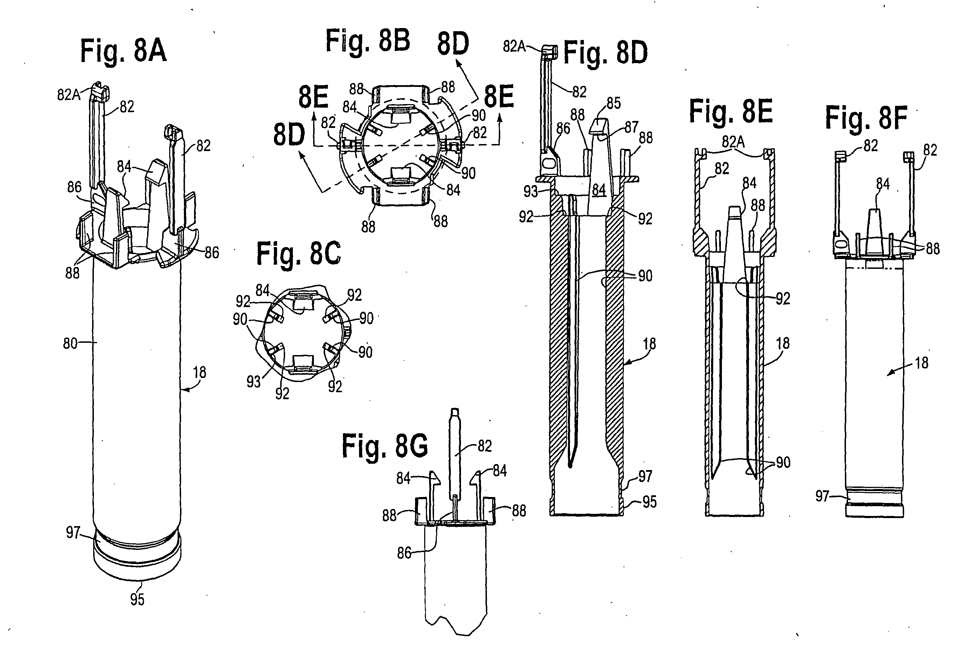

Figures 8A-8G are several views of the needle guard of Figures 1-4, and in particular Figure 8A is a perspective view, Figure 8B is a top end view, Figure 8C is a detail of the features at the top of the needle guard including the snaps which retain the flange of the syringe body within the device and the supports for the compression spring of Figure 4, Figure 8D is a cross-section of the needle guard along the lines 8D-8D of Figure 8B, Figure 8E is a cross-section of the needle guard along the lines 8E-8E of Figure 8B, Figure 8F is a side elevation view of the needle guard, and Figure 8G is another side view of the upper (proximal) portion of the needle guard.
Figures 9A-9E are several views of the inner tamper evidence overcap of Figures 2-4, and in particular Figure 9A is a perspective view, Figure 9B is a side view, Figure 9C is a cross-sectional view along the lines 9C-9C of Figure 9B, Figure 9D is an end view, and Figure 9E is an opposite end view.
Figures 10A-10F are several views of the outer tamper evidence overcap of Figures 1-4, and in particular Figure 10A and 10B are perspective views, Figure 10C is a side view, Figure 10D is another side view partially in cross-section, Figure 10E is a cross-section along the lines 10E-10E of Figure 10D, and Figure 10F is a detail of the inner corner of the outer tamper evidence overcap showing a pocket feature which is used to retain the inner tamper evidence overcap of Figure 9A-9E to the outer tamper evidence overcap.
Figure 10G is a perspective view of the washer feature of Figures 2-4 which is placed between the inner and outer tamper evidence overcaps during assembly. When the tamper evidence overcap is removed from the handle, the washer of Figure 10G pulls the needle sheath off the needle to make the needle ready to give the injection.
Figure 11 is a side view showing the removal of the tamper evidence overcap.
Figure 12 is a cross-sectional view of the injection device of Figure 11. Figure 12A is a detail view of the cross section of Figure 12 showing a strut within the handle which limits travel of the syringe body during removal of the overcap. Figure 12B is a detail view of the overcap showing a feature which prevents re-installation of the tamper evidence overcap onto the needle guard once the overcap has been removed.
Figure 13 is a side view of the injection device of Figures 1-4 after the tamper evidence overcap as been removed, showing the device ready for use to give an injection at an injection site. Figures 13A and 13B are cross-sectional views of the device of Figure 13.
Figure 14 is a side view of the device of Figures 1-4 showing the movement of the handle down over the needle guard in the process of administration of an injection at the injection site, such action causes the plunger to advance within the syringe body to expel medicament from the syringe. Figure 14A is a cross-section of the device of Figure 14. Figure 14B is a detailed view showing the compression of the compression spring between the syringe flange and the needle guard shoulder seat.
Figure 15 is a side view of the device of Figures 1-4 showing the further movement of the handle down over the needle guard in the process of administration of an injection at the injection site. Figure 15A is a cross-section of the device of Figure 15. Figure 15B is a detailed view showing track follower following a track in the plunger in the action of Figure 15.
Figure 16 is side view of the device of Figures 1-4 with the handle at its lowest position relative to the needle guard and plunger, at the point where the expelling of the medicament in the syringe is complete. Figure 16A is a cross-sectional view of the device of Figure 16. Figure 16B is a detailed view of the plunger track and track follower of Figure 16A.
Figure 17 is a side view of the device of Figures 1-4 with the handle at its lowest position relative to the needle guard and plunger, after the expelling of the medicament in the syringe is complete and the user removes the injection device from the injection site. Note that the needle shield has advanced beyond the tip of the needle to prevent against an accidental needle stick. Figure 17A is a cross-sectional view of the device of Figure 17. Figure 17B is a detailed view of the plunger track and track follower of Figure 17A.
Figure 18 is a side view of the device of Figures 1-4 after removal of the injection device from the injection site. The needle guard has advanced further to a locked position covering the needle. Figure 18A is a cross-sectional view of the device of Figure 18. Figure 18B is a detailed view of the plunger track and track follower of Figure 18A, with the track follower positioned within a locking pocket in the track.
Figure 19 is a perspective view of an optional spring support bushing.
Figure 20 is a cross section of the injection device with the bushing of Figure 19 installed inside the compression spring.
Figure 21 is a perspective view of another optional spring support bushing.
Figure 22 is a cross section of the injection device with the bushing of Figure 21 installed over the compression spring.
Figure 23 is an elevational view of a plunger adapted for use with a syringe which is less than fully filled, in which the track is positioned lower on the upper (proximal) portion of the plunger.
Figure 24A is a perspective view of an alternative embodiment of the plunger with a feature at the lower end thereof which grips the interior of the syringe so as to prevent axial travel of the syringe relative to the plunger when the overcap is being removed. Figure 24B is an elevational view of the plunger of Figure 24A. Figure 24C is a detailed view of the lower portion of the plunger of Figures 24A and 24B. Figure 24D shows the plunger of Figures 24A-24C positioned within the syringe showing the feature at the lower end of the plunger gripping the interior walls of the syringe.

### DETAILED DESCRIPTION

### Overview

Figure 1 is a side elevation view of an injection device 8 according to an embodiment of the invention. The device 8 includes a tamper evidence overcap 20 and a hollow tubular handle 10 which is grasped by the user to make an injection. Figure 2 is a cross-sectional view of the injection device 8 of Figure 1. Figure 3 is another cross-sectional view of the injection device 8 of Figure 1 taken in a plane orthogonal to the plane of Figure 1. Figure 4 is an exploded view showing all the parts of the device, including a plunger 12, a pre-filled syringe assembly 26, a compression spring 16, the hollow generally cylindrical handle 10, a hollow tubular needle guard 18 having track followers 82, an inner overcap 22, a washer 24 and the outer overcap 20.

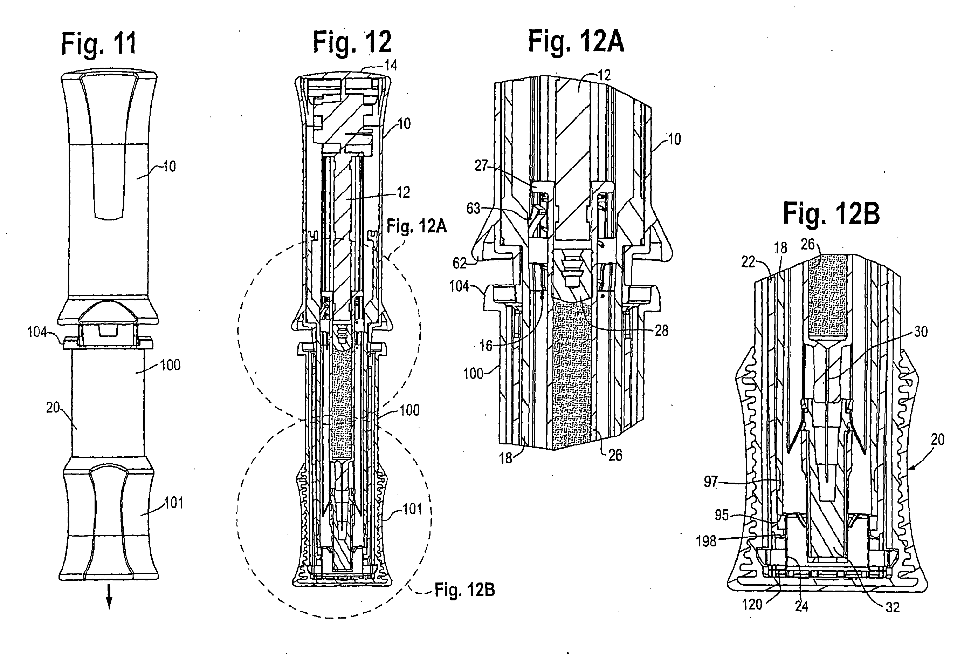

The discussion will proceed to first describe the construction of the device 8 and several significant features thereof, in conjunction with Figures 1-11. A step-by-step explanation of the manner of using the device 8 to administer an injection to a human or animal subject will be described in conjunction with Figures 12-18 later in this document. It will be appreciated that the following discussion, including advantages and features of the illustrated embodiment is offered by way of illustration and not limitation. All questions regarding the scope of the invention are to be answered by reference to the appended claims.

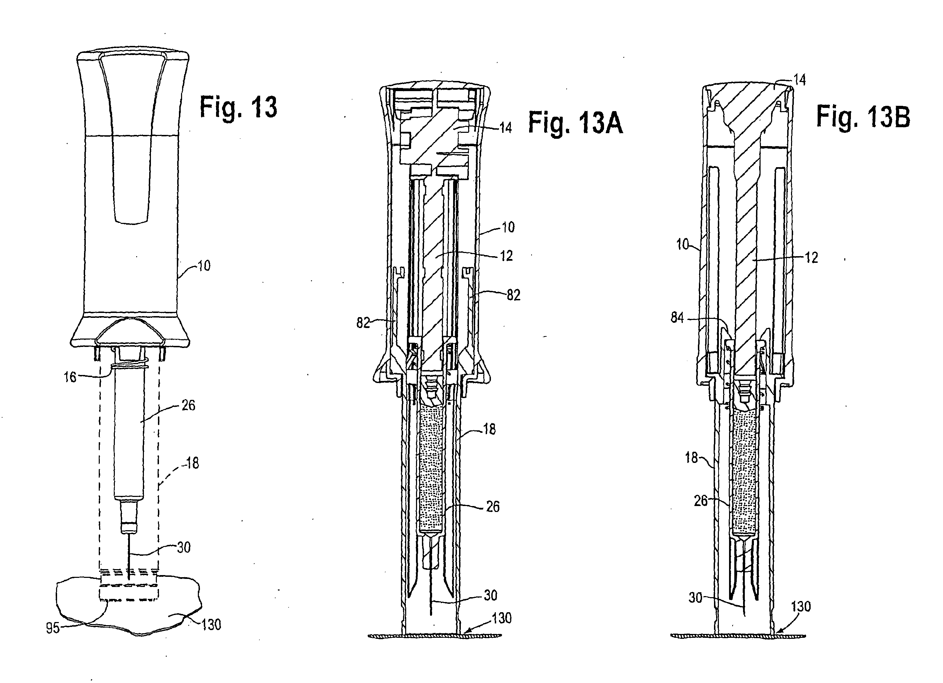

The injection device 8 is constructed to receive a pre-filled syringe 26 and administer and injection of medicament contained within the pre-filled syringe to a human or animal subject. The design is particularly suitable for self-administration of injections, such as for example self-administration of insulin or arthritis medication. The device includes a hollow tubular needle guard 18 which surrounds the needle 30 of the pre-filled syringe 26. The needle guard 18 and a plunger 12 for the syringe 26 are located within the handle 10. The handle 10, the needle guard 18 and the pre-filled syringe 26 move axially in relation to each other. In the final assembled device, the pre-filled syringe 26 sits within the needle guard 18 and is able to move axially relative to the needle guard 18 and handle 10. Relative to the user (patient), the handle 10 is proximal and held in the hand. The needle guard 18 is distal and represents the portion of the device that is placed against the injection site after the tamper-resistant overcap 20 is removed, as shown in Figure 13, 13A and 13B.

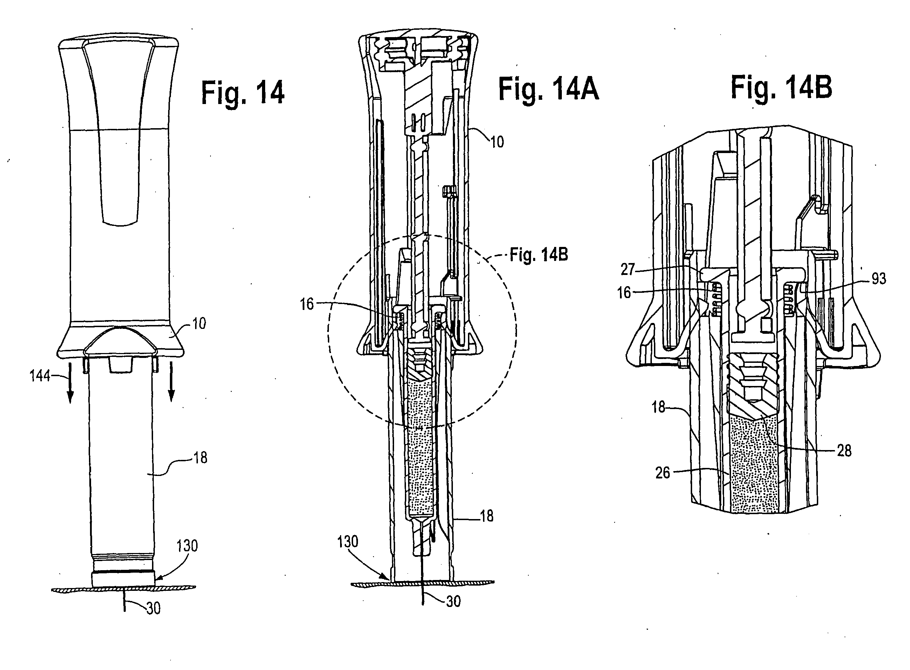

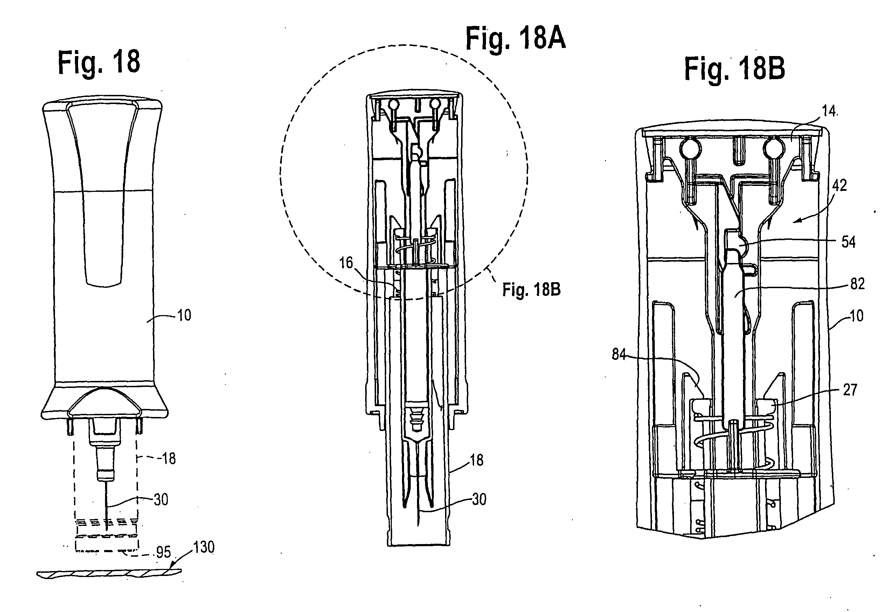

The plunger 12 includes a top portion 14 which is attached to the open proximal end of the generally cylindrically-shaped handle 10. As the handle 10 is moved towards the injection site during an injection (as will be explained more fully below in conjunction with Figures 14-16), the handle moves the plunger 12 tip 15 through the interior of the pre-filled syringe 26 to expel medicament from the pre-filled syringe through the needle 30. At the completion of the injection and as the device is removed from the injection site, the needle guard 18 is moved distally by a spring 16 and locked into a position to cover the tip of the syringe needle 30 to prevent an accidental needle stick (Figure 18).

The preferred embodiment of the injection device 8 is believed to possess several distinct advantages. First, it provides an ergonomic, easy-to-use device that is particularly suitable for self administration of injections by patients with dexterity limitations, including the elderly and persons suffering from arthritis in the hands or fingers. The handle 10 is preferably designed without sharp edges or surfaces that would generate pressure points during use. No squeezing is required. The wide tubular structure of the handle allows for ease of grip by the user, in a variety of different hand positions. The injector handle 10 is designed to provide a stable hand grip.

The needle guard 18 provides a stable injection site because it is wide enough at the point of contact with the skin. It is also preferably designed with no sharp edges at its distal surface, in the area where the needle guard makes contact with the injection site.

Completion of the injection, as will be explained in Figures 12-18 subsequently, is not technique-dependent. The device includes several features which minimize the chance of injection error. The design includes features to control the depth of the needle penetration. There is no need for an angled administration of the injection; rather the injection is administered with the injection device oriented perpendicular to the injection site. There is no need for the patient to pinch or gather the skin at the injection site.

The design of the injection device 8 also includes several features to minimize patient anxiety. The patient does not need to see the needle 30 to achieve injection. The possibility of inadvertent needle stick .prior to initiation of the injection is limited because the injector includes a needle guard feature (needle guard 18) which covers the needle 30 prior to the injection. Furthermore, the patient does not need to worry about re-capping the needle 30 after use, or an accidental needle stick after use, as the device automatically locks the protective needle guard (needle guard 18) in an extended position to cover the tip of the needle 30 at the completion of the injection.

The design further reduces hazards from accidental breakage of a glass syringe 26 containing the medicament. The syringe 26 is enclosed in plastic (by virtue of being housed within the both the needle guard 18 and the handle 10), thus is protected from impact and bending stresses. Additionally, the handle and the tamper evidence overcap are both preferably provided with flats (flat surfaces) which prevent the device from rolling when placed on a hard horizontal surface, thereby reducing the likelihood that the device will roll off a table onto the floor and breaking or damaging the syringe 26 or needle thereof.

Furthermore, the needle 30 tip is protected from coming into contact with surfaces prior to initiation of the injection, reducing the risk of contamination due to contact with non-sterile surfaces, and reducing the possibility of damage or dulling of the needle tip, thereby reducing the likelihood of a painful injection due to a dull needle tip.

An additional feature of the device 8 is that it includes a tamper evidence overcap 20. Should the tamper evidence overcap 20 be removed from the device (e.g., in the case of tampering) the overcap 20 cannot be readily re-installed onto the needle guard 18 of the injector device 8. Thus, if the user sees the tamper evidence cap 20 in place on the device the user is reasonably assured that the device has not been tampered with. Other noteworthy aspects of the tamper evidence overcap 20 are that it is designed such that neither a twisting motion, nor a peeling motion, is required to remove the overcap, unlike many other tamper evidence structures. This makes the design easier to use, particularly for users with limited hand mobility or strength. The removal of the overcap does not require any special tools such as scissors or a knife. There are no plugs or other devices to remove. Rather, the user simply pulls the overcap 20 off and the device is ready for immediate use.

The cross-section of Figure 2 shows the assembled injection device 8 including the tubular handle 10 and the plunger 12, with the overcap 20 in place. The plunger 12 includes a top portion of the plunger 14 which is secured to the open top of the handle 10, and a bottom edge 15 which seats on a stopper 28 positioned within the pre-filled syringe 26. A helical coil spring 16 is positioned surrounding the pre-filled syringe 26. The top edge of the spring 16 seats against a distal portion of the flange 27 formed on the upper edge of the pre-filled syringe 26 (see Figure 6). The bottom edge of the spring 16 seats on a ledge portion 92 of four ribs 90 projecting into the interior of the hollow tubular needle guard 18 (See Figures 8B, 8C and 8D). Prior to placing the device 8 on the injection site, the force of the spring 16 maintains extension of the needle guard 18 in a position beyond the tip of the needle 30. When the device is applied to the injection site, with some pre-determined minimal force overcoming the spring force of the spring 16, the needle guard 18 moves proximally (i.e., is retracted) allowing advancement of the needle into the injection site.

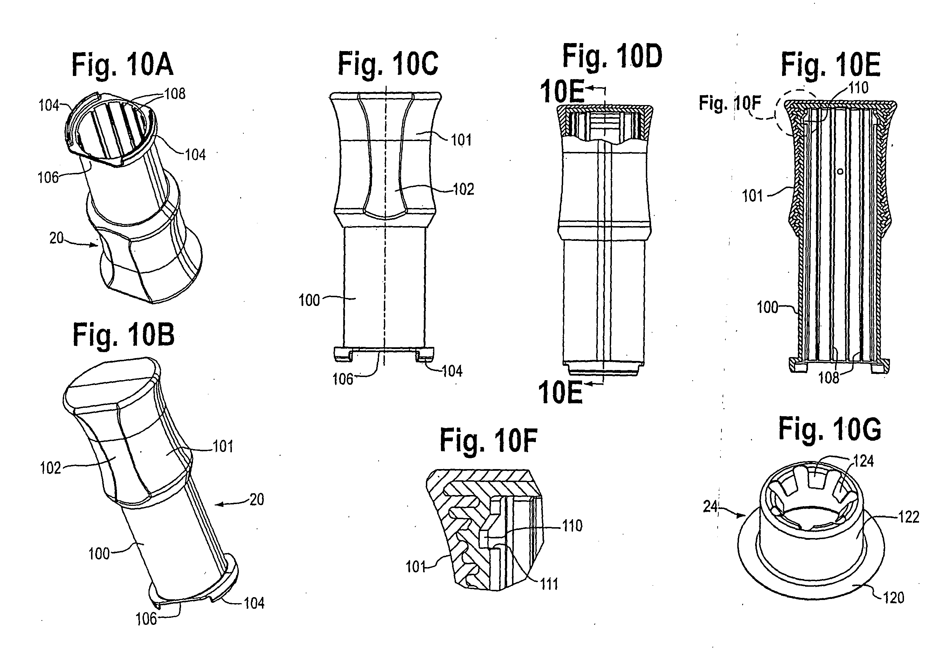

In the assembly of Figures 1-4, the needle 30 is protected by a needle sheath 32 prior to removal of the tamper evidence overcap 20. The overcap 20 includes a feature to grip the needle sheath 32 and pull the needle sheath 32 off of the needle 30 at the same time as the overcap 20 is removed from the handle and retain the needle sheath 32 securely within the overcap 20. While several possible constructions to achieve this function are possible (depending on such factors as the design and materials of the needle sheath 32), in the illustrated embodiment the feature takes the form of a hat-shaped retainer 24 which is positioned and retained within the overcap 20. The retainer 24 grips the needle sheath 32. When the overcap 20 is removed, the retainer 24 pulls the needle sheath 32 off of the needle 30. In one possible configuration, the needle sheath 32 is made from a resilient material such as plastic and the retainer 24 is made from a material that is at least as rigid, or more rigid, than that of the needle sheath, such as metal or hard plastic. The fingers 124 of the retainer 24 (Figure 10G) are tapered slightly inwardly to allow the needle sheath to be inserted into the retainer 24 but prevent withdrawal of the sheath from the retainer. The fingers 124 frictionally engage the needle sheath 32 when the device is assembled. The fingers 124 (Figure 10G) pull the needle sheath 32 off the needle and hold the sheath 32 securely within the overcap 20 as the tamper evidence overcap 20 is removed from the handle 10. After removal of the overcap from the handle, the needle sheath will not come loose from the overcap due to the gripping of the sheath by the fingers 124 of the retainer 24. The retainer 24 is preferably dimensioned such that it can float slightly, both axially and transversely, relative to the inner tamper evidence overcap. This design permits the syringe to be fully inserted into the overcap during assembly of the injection device such that the needle sheath is reliably inserted into the space between the fingers 124 of the retainer 24 and thereby securely gripped by the fingers 124 as shown in Figure 2.

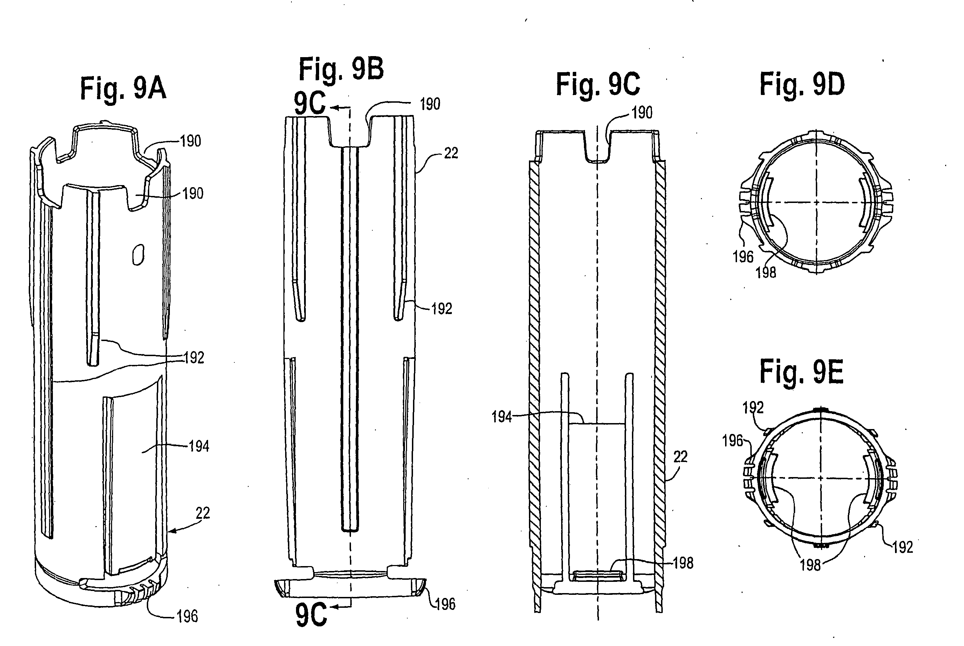

The overcap 20 is constructed with an inner overcap 22 (Figures 2-4) secured to the outer overcap 20. The inner overcap 22 functions as a tamper evidence feature to prevent re-installation of the overcap 20 over the handle 10 and needle guard 18. The inner overcap 22 also functions to retain the retainer 24 within the outer overcap 20. The construction of the inner overcap 22 will be explained below in conjunction with Figure 9.

The pre-filled syringe 26 is supported radially within the interior of the needle guard 18. Axial motion of the syringe 26 is limited in the distal direction by a needle guard feature (ledge 93 in Figure 8D) that serves as a stop limiting movement of the syringe barrel flange 27 (Figure 6). The pre-filled syringe 26 interacts with the handle 10 by way of the plunger 12. The bottom end 15 of the plunger 12 seats on the top surface of the syringe stopper 28. The top end 14 of the plunger 12 snaps onto the top of the handle 10. Note that the plunger 12 does not need to be a separate component. Rather, it could be integrated as a feature of the handle 10. Furthermore, the plunger could be constructed as two pieces, namely a top piece that is either a separate component or part of the handle, and a bottom piece (rod) that could be part of the syringe or the syringe stopper. Additionally, the lower tip 15 of the plunger 12 could be designed to snap or screw on to the stopper of the syringe. In these various other configurations the essential functioning of the injection device is unchanged.

As will be explained further in following sections, at the start of injection, the needle guard 18 is retracted relative to the needle and the needle 30 is sub-cutaneously inserted into the injection site, and the syringe stopper 28 (Figure 2) is in a retracted, proximal position. As the user applies the downward injection force on the handle 10, the force is transmitted from the handle, through the plunger 12, to the stopper 28, causing the stopper to advance within the body of the pre-filled syringe 26 and delivering the medicament from the syringe 26. In the course of this advancement, the handle 10 slides down (and over) the needle guard 18, gradually bringing their proximal ends into closer proximity (See Figures 14-16).

The discussion will now turn to a detailed description of the components of the illustrated embodiment.

### Plunger 12 (Figures 5A-5D)

The plunger 12 of Figures 2-4 is shown isolated in Figure 5A-5D, with Figure 5A being an end view, Figure 5B being a side view, Figure 5C being another side view at a 90 degree angle from the side view of Figure 5B, and Figure 5D being a perspective view. As shown best in these Figures, the plunger 12 includes a lower tip 15 which seats on the top end of the stopper 28 within the syringe 26, an upper portion 14 which includes two tracks 42, and pair of flexible tabs 40 which engage features 60 (Figure 7A, 7B) in the handle 10 to secure the plunger 12 to the handle 10 at the time of assembly of the device. The tabs 40 also serve as a tamper-evidence feature in that they serve to prevent removal of the plunger from the handle and thus prevent access to the syringe after the injection device is assembled. The length of the tabs 40 in the radial direction may vary, with longer tabs serving to provide deeper penetration into adjacent structure in the handle and make it more difficult to remove the plunger from the handle. Furthermore, the domed surface to the top of the handle (seen best in Figure 5B and 5C) is flush to adjacent structure in the handle 10 (as best seen in Figure 2 or Figure 3), which further makes tampering with the device more difficult because it prevents insertion of a tool between the handle and the plunger in order to remove the plunger from the handle.

The plunger 12 includes two tracks 42 (Figures 5A and 5D), one for each of the track followers 82 of the needle guard (Figure 4, 8A). The term "track" is intended to be construed to mean any physical structure, whether characterized by walls, grooves, slots, or combination thereof, which serves a function to guide the travel of another part, the "track follower" herein. Except as noted below, the two tracks 42 have the same design and configuration, which is shown best in Figures 5C and 5D, therefore only one will be described. The tracks 42 are defined by walls including a ramp 44 which guides the track follower 82 to the left at the beginning of the injection, a vertical wall portion 46, an open region 48 above the wall 46, a rib 50 extending into the open region 48, a second ramp 52 to guide the track follower to the right after completion of the injection, a corner feature 56 and a locking pocket 54. The track follower 82 is retained in the lock pocket 54 by the corner feature 56. The locking of the track follower 82 in the locking pocket 54 serves to hold the needle guard 18 (integral with the track follower) in an extended position at the end of the injection to cover the tip of the needle 30.

As will be now appreciated from Figures 5C, and 15-18, the proximal ends of the needle guard 18 and plunger 12 incorporate features (the track follower 82 and the track 42) that contact each other and react in a manner that allows the needle guard 18 to lock into an extended position at the completion of injection. The configuration of these reacting features is such that the lockout will not be enabled unless (until) the full dose is delivered (i.e.; plunger 12 moves to the distal end of pre-filled syringe body 26).

The structure and operation of the track 42 and track follower 82 (Figures 2, 4 and 8A) will now be described in greater detail. Briefly, in the illustrated embodiment, the track follower 82 of the needle guard 18 is an elongate flexible finger-like feature that extends axially and embodies a hooked end at an approximate 90° angle. The mechanical properties of the track follower 82 are configured to allow a flexible, cantilevered deflection in multiple planes. The track feature 42 of the plunger 12 is essentially a channel or groove defined by the walls and features 44, 46, 48, 52, 54 and 56 that runs mostly axial (lengthwise) along the surface of the plunger top 14, best shown in Figures 5C and 5D. The track follower 82 follows the channel of the track feature 42. In the illustrated embodiment, the track 42 is created by the providing walls of a predetermined configuration in the plunger top 14. An alternate embodiment of the track principle could utilize a recessed groove. The walls of the track 42 are intended to capture and guide the hooked end of the track follower 82 along a mostly axial path but with some side-to-side deflection at specific points in travel due to flexibility of the track follower. The bottom portion of the track 42 is mostly parallel to the long axis of the plunger but employs an abrupt transition to create a pocket (54 in Figure 5C) that captures the hooked end of the track follower 82 after the injection is complete and the injection device is withdrawn from the injection site.

As the injection process proceeds, the track follower 82 will engage with the ramp 44 and be deflected to the left. After engagement, as the needle guard 18 and handle 10 continue relative coaxial motion, the hook of the track follower 82 traverses the track 42 along a path to the left of the wall 46 which guides it along a mostly axial path but with a slight deflection to the left (due to wall 46 being slightly off-center). This side deflection creates a mild force potential as the flexible properties of the track follower 82 cause it to resist the deflection. At the bottom of the injection stroke, the side walls of the track 42 are configured to allow the track follower 82 to enter the open region 48 (Figure 5C) and move back into the original relaxed position. To do so, the track follower will contact and move past the rib 50, making an audible click noise to signal to the user that the injection of medicament is substantially complete. (Note that in the illustrated embodiment only one of the two tracks 42 includes the rib 50 in order to prevent a possible double click. However, a rib 50 could be provided in both tracks). At the point of this new position (track to the right of the rib 50), the track 42 walls are configured to guide the hook of the track follower 82 along a new channel in the track, that is, down the ramp 52. Following injection, when the user lifts the device from the injection site, the needle guard 18 is allowed to advance forward (distally) in reaction to the force of the compression spring 16. The advancement of the needle guard 16 is also relative to the handle 10, meaning that the track follower 82 will now follow along the track 42 down the ramp 52. At the point where the needle guard is again extended over the tip needle, the track features a corner 56 which terminates the bottom of the ramp 52. The flexible characteristics of the track follower cause the hook of the track follower to slide past the end of the corner 56 and to move into a lock pocket 54 (Figure 5C). When the track follower 82 is in the lock pocket 54, the needle guard 18 cannot be retracted within the handle 10, thus maintaining its position covering the needle tip and preventing an accidental needle stick after completion of the injection. The lock pocket 54 or corner 56 may also be designed such that a second "click" occurs when the track follower slides past the corner 56 into the lock pocket 54 to signal to the user that the lock-out has occurred.

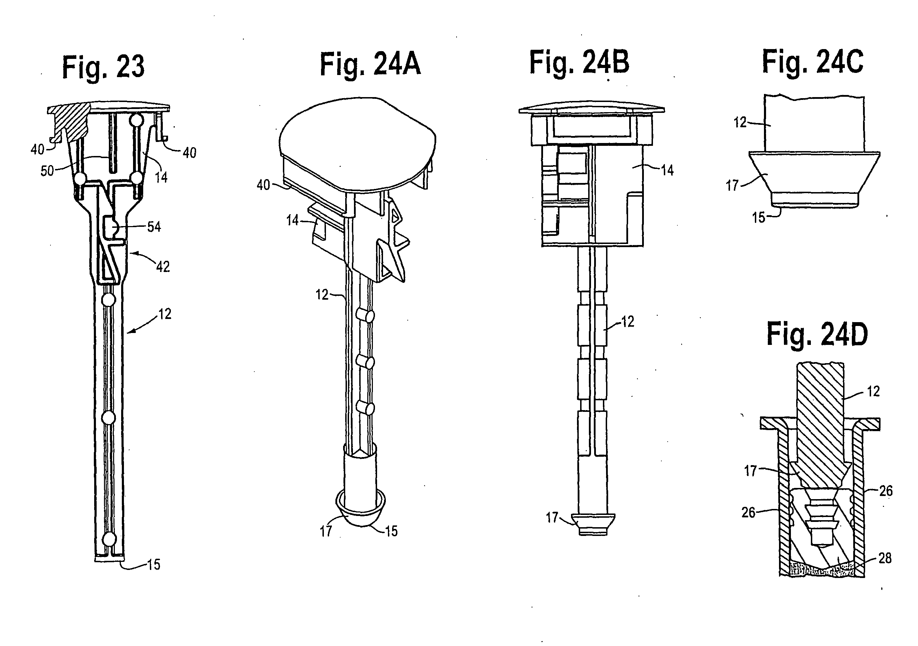

The axial length of the plunger can vary in order to inject a different dose from the syringe. For example, a syringe may be provided which is only half filled with medicament in order to administer a smaller dose. In this case, the stopper may be positioned initially roughly in the middle of the syringe body. To accommodate this change, the plunger will be made longer in axial length such that the lower tip 15 of the plunger will contact the upper surface of the stopper deeply within the interior of the syringe when the device is assembled. In this situation, assuming no change is made to the design of the needle guard and track follower thereof, the tracks 42 are formed the plunger in a different location than shown in Figure 5C. In particular, the tracks are moved in the distal direction by the amount equal to the added length to the plunger rod, as shown in Figure 23.

### Pre-filled Syringe Assembly (Figure 6)

The pre-filled syringe assembly is shown in Figure 6 in an exploded view and includes a tubular syringe body 26 having an open interior for containing a medicament, a flange 27, a stopper 28 placed within the syringe body 26, a needle 30, and a needle sheath consisting of an inner sheath 32A and a sheath cover 32B which fits over and is secured to the inner sheath 32A. A one-piece needle sheath is also possible. The sheath cover 32B can be made from a variety of materials such as plastic or metal. The bottom tip 15 of the plunger 12 (Figure 5B) seats on the top surface of the stopper 28. The plunger 12 serves as the implement to advance the stopper 28 through the interior of the syringe 28 to expel medicament through the needle 30. As noted previously the plunger tip 15 could be provided with features to snap or screw on to the stopper. In one possible embodiment, the syringe 26 is made from clear glass.

The type of medicament contained within the syringe 26 is of course not particularly important. The medicament could for example take the form of insulin, arthritis medication, allergy medication, etc.

### Handle 10 (Figure 7A-7G)

The handle 10 of Figures 1-4 is shown isolated in Figure 7A-7G. The handle 10 is a generally hollow, tubular body with open ends. Figure 7A is a perspective view showing a flat portion 62 formed on both sides of the handle to prevent the handle from rolling when it is placed on a hard surface. The flat portions 61 at the lower end are used to align the handle 10 with the overcap 20 during assembly.

The handle includes a pair of ledges 60, best seen in Figures 7A-7E, the lower edge of which engages with the resilient snap features 40 of the plunger 12 (Figure 5B) to retain the plunger 12 to the handle 10 when the device is assembled. As shown best in the cross-section of Figure 7C, the interior of the handle 10 includes a set of raised ridges 64 which serve to align the needle guard 18 (and particularly the track follower thereof) to the plunger and track features thereof during assembly. The short raised rib features 65 at the top of the handle provide support for the four wall portions 57 of the plunger (see Figure 5A, 5C and 5D). Figure 7E is a detail of the ledge feature of Figure 7D and also showing the rails 64 which guide insertion of the needle guard 18 into the handle 10 during assembly and prevent axial rotation of the needle guard 18 relative to the handle.

The lower end of the handle 10 includes a pair of generally inclined, upwardly and inwardly directed struts 63 which project into the interior of the handle 10. The purpose of the struts 63 is to limit the amount of axial movement of the syringe 26 in the distal direction during the removal of the overcap 20 and needle sheath 32, as will be explained later in conjunction with Figures 12 and 12A. Basically, the struts 63 prevent a "slingshot" effect due to excess compression of the spring 16 during removal of the overcap and needle sheath 32. Release of the spring force from such compression would cause the syringe to spring back against the underside of the needle guard snaps 84, which would produce an unwanted audible and tactile feedback to the user. To prevent this effect, the struts 63 limit the axial movement of the syringe flange in the distal direction and allows minimal compression of the spring 16 during removal of the overcap. Referring to Figures 24A-24D, an alternative to this design is to incorporate a flared feature 17 in the bottom portion of the plunger 12 which grips the interior walls of the syringe 26 and thereby resists movement of the syringe relative to the plunger in the axial direction. When the overcap is pulled off, and with it the needle sheath, friction between the walls of the flared feature 17 and the interior of the syringe 26 prevents movement of the syringe 26 relative to the plunger 12, preventing the "slingshot" effect. The flared feature 17 is oriented as shown in Figure 24B and 24C such that only insignificant additional resistance is created when the plunger tip 15 advances within the syringe 26 to expel the medicament.

The lower end of the handle 10 also includes a rim 60 and a recessed region 70 which receives the rim 104 (Figure 10B) of the outer tamper evidence overcap 20. The handle 10 further includes four projections 66 which are received in four complementary recesses 190 (Figure 9A) in the inner overcap 22 when the device is assembled.

### Needle guard 18 (Figure 8A-8G)

The needle guard 18 of Figures 2-4 is shown isolated in various views in Figure 8A-8G. The purpose of the needle guard 18 is to function as a protective guard for the syringe needle 30 both before and after an injection. Figure 8A is a perspective view of the needle guard 18. The needle guard includes a generally hollow, tubular cylindrical body 80. Extending from the cylindrical body 80 are two elongate flexible finger-like features 82 which server as track followers for the tracks 42 in the plunger. The track followers 82 have hooked ends 82A which engage the track 42 (Figure 5C) of the plunger 12. The track followers 82 extend from supports 86 as best shown in Figures 8A, 8D and 8G.

Referring to Figures 3 and 8A, the needle guard 18 further includes a pair of snaps 84 which flex outwardly to allow the syringe 26 to be inserted into the interior of the cylindrical body 80 and the flange 27 to pass down the slanted surface 85 (Figure 8D) of the snaps 84 and flex the snaps 84 outwardly. After the syringe flange 27 has passed the snaps 84, the lower edge 87 of the snaps 84 prevents axial movement of the flange 27 of the syringe in the proximal direction. Travel of the syringe 26 in the distal direction is limited by a shoulder 93 (Figure 8C and 8D). The interior of the needle guard 18 includes raised ridges 90 which support the walls of the syringe 26. The shoulder 92 of the ridges 90 provides a seat for the lower end of the coil spring 16 (Figure 4).

The needle guard further includes opposed pairs of walls 88 which are spaced from each other such that a pair of the walls 88 fit within the rails 64 formed on opposite sides of the interior surface of the handle (see Figures 7C, 7E and 7F) in order to maintain proper alignment of the needle guard and track followers 82 to the track 42 of the plunger.

The lower portion of the needle guard 18 includes a recessed feature shown as a ring 97 which receives the ledge 198 at the end of a resilient finger 194 which project inwardly from the lower end of the inner overcap 22 (see Figure 9C). After removal of the tamper evident overcap 20/22, the ledges 198 prevent the inner overcap 22 from being reinserted onto the needle guard 18 due to interference between the ledges 198 and the end wall 95 of the needle guard 18. The recessed feature 97 could take other forms, such as a pair of opposed pockets or voids in the surface of the needle guard in alignment with the ledges 198.

The needle guard 19 may be made from any suitable material such as plastic, either transparent or opaque. The use of a clear, transparent material such as clear plastic for the needle guard allows the user to see the syringe 26 within the needle guard 18 after removal of the tamper evidence overcap 20.

### Tamper Evidence Overcap 20 (Figures 1-3, 9A-9E, 10A-10F)

The tamper evidence overcap of Figures 1-3 will now be further explained. The tamper evidence overcap includes an inner overcap 22 (Figures 9A-9E) and an outer overcap 20 (Figures 10A-10F. The overcap further includes a feature (retainer 24 of Figure 4) which functions to remove the needle protective sheath 32 at the time of removal of the overcap.

### A. Inner overcap 22 (Figures 9A-9E)

The inner overcap 22 is shown isolated in Figures 9A-9E. The inner overcap 22 is a generally hollow tubular body with open ends, and includes recessed portions 190 which mate with the projections 66 on the bottom of the handle 10 (see Figure 7B) when the overcap is placed onto the handle 10. The inner overcap also includes guide rails 192 which guide the insertion of the inner overcap into the interior of the outer overcap 20 between rails 108 (Figure 10E) in a predetermined manner such that the resilient projections 196 of the inner overcap fit into and engage pockets 110 (Figure 10E, 10F) in the outer overcap 20 to retain the two pieces 20 and 22 in an assembled condition. As the inner overcap 22 is inserted into the outer overcap 20 during assembly, the resilient projections 196 flex inwardly to allow insertion into the outer overcap 20 and when the projections 196 reach the pockets 110 in the outer overcap the projections extend to their unconstrained, natural position within the pockets 110 and interfere with the lip 111 of the pocket 110 to prevent removal of the inner overcap 22 from the outer overcap 20.

The inner overcap 22 also includes a flexible cantilevered finger 194 having a ledge 198 at the lower end thereof. When the tamper evidence overcap 20/22 is installed, the ledge 198 fits in the recessed feature 97 (Figure 8A) of the needle guard. However, when the tamper evidence overcap 20/22 is removed from the needle guard, the ledges 198 interfere with the end wall 95 of the needle guard and prevent the overcap 20/22 from being reinstalled onto the needle guard 18, serving a tamper-evidence function.

### B. Outer overcap 20 (Figure 10A-10F)

The outer overcap 20 is shown isolated in Figures 10A-10F. The outer overcap 20 includes a proximal portion 100 and a distal portion 101 which is grasped by the user to remove the overcap 20. The overcap includes flat portions 102 which are aligned with the flat portions 62 of the handle (Figure 7A) to prevent the injector device from rolling on a hard surface. The proximal portion includes a raised rim 104 which fits within the region 70 (Figure 7B) of the handle 10 when the device is in an assembled condition with the overcap 20 installed. The edge region 106 is aligned with the flat portion 61 (Figure 7A) of the handle 10. Figure 10E shows the raises ribs on the interior of the overcap 20 which guide insertion of the inner overcap 22 into the outer overcap 20 at the time of assembly. The outer overcap 20 includes the pocket 110 in its distal portion to receive the resilient elements 196 of the inner overcap 22 (Figure 9D) to retain the inner and outer overcaps in an assembled condition.

The outer overcap 20 can be molded from a single piece of plastic, or constructed in a multiple shot molding process resulting in multiple layers of plastic as shown by the hatching in Figure 10E. The outer overcap 20 could also be constructed from separate pieces which are affixed together.

### C. Needle shield-engaging retainer 24 (Figure 10G)

In a preferred embodiment, the overcap assembly includes a feature to automatically remove the needle sheath 32 (Figures 2-4) from the needle 30 at the same time as removal over the overcap 20/22. The feature in the illustrated embodiment takes the form of a hat-shaped retainer 24 which is retained within the overcap between the inner and outer overcaps 20 and 22. The retainer 24 is shown in Figure 10G in a perspective view and includes a rim 120, a tubular body portion 122 and a plurality of fingers 124 which project slightly inwardly from the body 122 and which serve to frictionally engage the resilient needle sheath 32 in the manner described previously. In particular, during assembly the syringe and needle sheath is inserted into the overcap assembly. The needle sheath 32 and/or the fingers 124 are sufficiently resilient such that the sheath can be inserted past the fingers 124 with slight force. The fingers 124 frictionally engage the needle sheath 32. By virtue of the constraint of the retainer 24 between the inner and outer overcaps 22 and 20, respectively, when the overcap 20 is removed, the fingers 124 pull the needle sheath 32 off of the needle and hold the needle sheath 32 securely within the inner overcap. Consequently, when the overcap 20 is removed the injection device (and needle thereof) is ready for immediate use.

### 2. Method of Use

The method of using the device of Figures 1-11 will now be described with reference to Figures 11-18.

### A. Removal of Tamper Evidence Cap (Figures 11, 12A, 12B)

As shown in Figure 11, the user grasps the proximal end 100 and/or the distal end 101 of the outer tamper evidence overcap 20 with their fingers and pulls the cap in an axial direction as indicated by the arrow to remove the overcap 20 from the handle 10.

As shown in Figures 12 and 12A, as the cap 20 is being removed, the retainer 24, which is gripping the needle sheath 32, pulls the syringe 26 downwardly axially until the syringe flange 27 bottoms out on the handle strut 63. The downward motion of the syringe 26 is stopped by the strut 63. This limited travel of the syringe 26 in the distal direction during removal of the sheath 32 serves to prevent a snapping of the syringe back against the retaining snaps 84 of the needle guard when the sheath 32 completely disengages from the needle 30. Further travel of the cap 20 slides the tamper evidence resilient ledge 198 of the inner cap 22 past the end 95 of the needle guard 18, preventing the user from placing the overcap back onto the device due to interference between the ledge 198 and the end 95 of the needle guard 18 in the manner explained previously.

### B. Inspection of Drug and Placement at Injection Site (Figures 13, 13A, 13B)

Once the overcap has been removed, the user positions the injection device at an injection site 130 (Figure 13) with the lower edge 95 of the needle guard in contact with the skin. The clear needle guard 18 allows the user to inspect the syringe 26. As shown in Figures 13A and 13B, note that prior to administration of the injection the needle 30 is protected within the lower portion of the needle guard 18 to prevent accidental needle sticks or touching of the needle with surfaces which might contaminate it or dull the tip of the needle 30. The device is preferably placed on the injection site 130 and oriented at a 90 degree angle to the surface of the injection site.

### C. Puncture of Skin (Figures 14, 14A, 14B)

As shown in Figure 14, after placement of the injection device at the injection site 130 the user grasps the handle 10 and pushes down on the handle 10, applying force in the direction of the arrows 144. The handle 10 slides over the needle guard 18. As shown best in Figures 14A and 14B, the coil spring 26 compresses with minimal force by the user and the needle guard 18 retracts such that the needle 30 is exposed and is inserted into the skin. The depth of needle penetration into the skin is controlled by the contact between the syringe flange 27 and the shoulder 93 of the needle guard 18, seen best in Figure 14B.

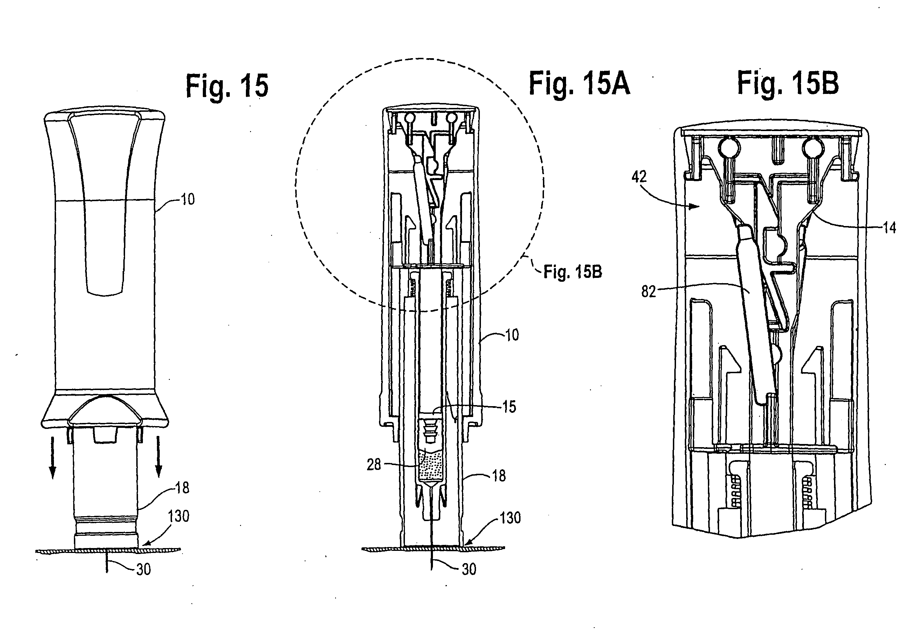

### D. Delivery of Medicament from Syringe Assembly (Figures 15, 15A, 15B)

Referring now to Figures 15 and 15A, continuous force on the handle 10 moves the handle 19 downward relative to the needle guard 18, thereby advancing the plunger 12 tip 15 and stopper 28 through the interior of the syringe 26 and expelling the medicament contained therein out the tip of the needle 30. Meanwhile, as shown in Figure 15B, the advancement of the plunger 12 guides and deflects the track follower 82 up on the left side of the track 42 on the upper portion 14 of the plunger 12.

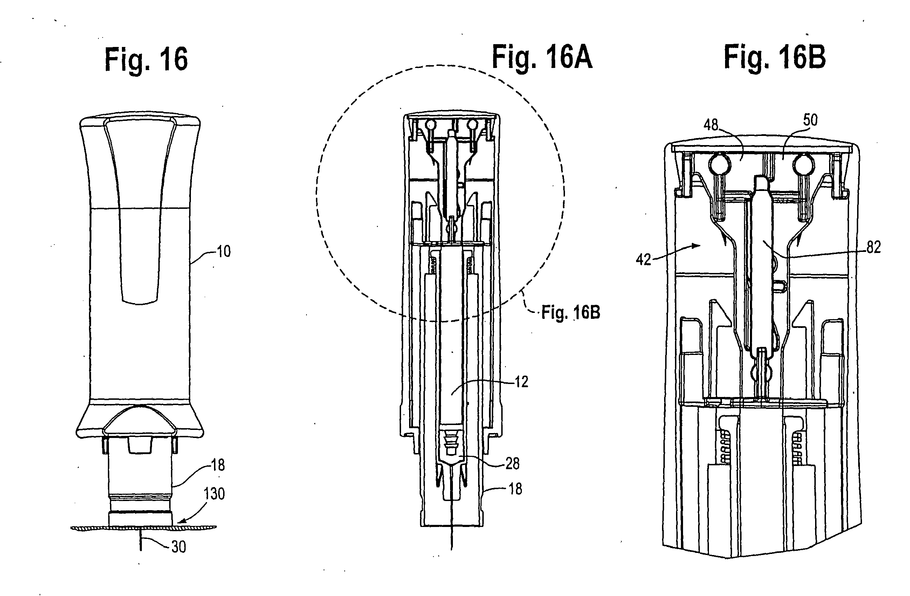

### E. Completion of Medicament Delivery (full dose) (Figures 16, 16A, 16B)

Referring now to Figure 16, the user continues to apply force on the handle 10 to complete the expelling of medicament from the syringe and needle 30. The user will feel a slight increase in resistance at the point when the injection is complete. The stopper 28 will be visible at the bottom of the syringe 26 (Figure 16A), particularly if the needle guard 18 is made from a clear material. Referring to Figure 16B, the track follower 82 moves to the top of the track (open region 48 in Figure 5C) and springs back to its natural center. In doing so, it passes over or against the rib 50 on the plunger 12 in the region 48, which results in an audible sound or "click" to the user indicating to the user that the injection of medicament is substantially complete.

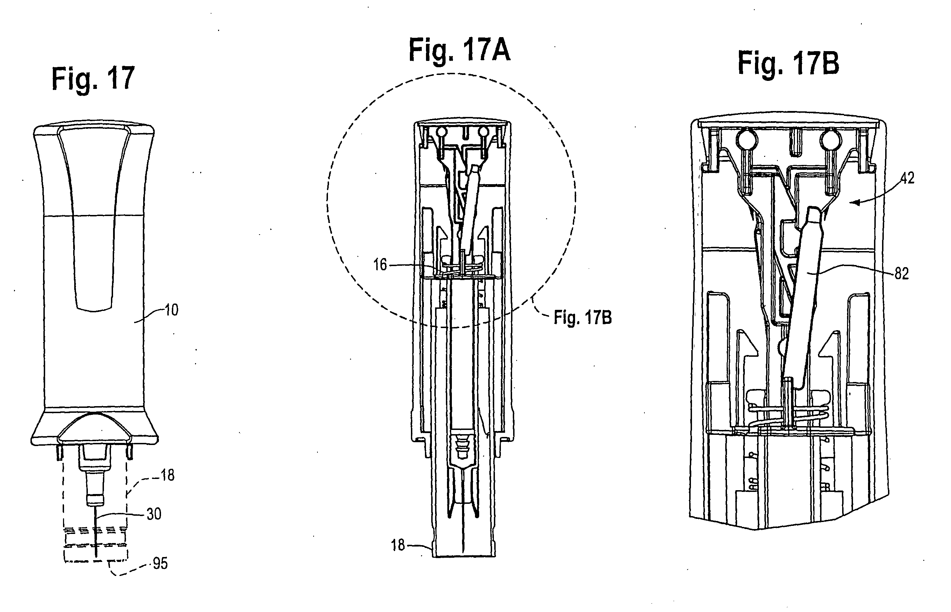

### F. Removal of Device from Injection Site (Figure 17, 17A, 17B)

Referring now to Figure 17, the user lifts the handle 10 off of the injection site. Force on the handle 10 relative to the needle guard 18 has been removed. Consequently, the coil compression spring 16 (Figure 16) moves the needle guard 18 relative to the handle 10 such that the lower rim 95 of the needle guard 18 covers the needle 30. Referring to Figure 17B, once the spring 16 begins to lengthen and the needle guard 18 advances forward relative to the handle 10 and plunger 12, the track follower 82 begins to slide down the right side of the track 42 and down the ramp feature 52 of Figure 5C.

### G. Device Lock-out (Figures 18, 18A, 18B)

Referring now to Figure 18, the coil spring 16 returns to its extended condition, the needle guard 18 motion in the distal direction relative to the handle 10 comes to an end and the needle 30 is recessed within the needle guard 18 as shown. Referring to Figure 18A and 18B, the motion of the coil spring 16 returning to its extended condition and exerting force on the needle guard advances the track follower 82 down the right side of the track 42 until the track follower 82 advances past the corner feature 56 (Figure 5C), whereupon the resiliency of the track follower restores the track follower to a centered position, such that the track follower 82 moves into the lock pocket 54 (Figure 5C) of the track 42. Once the track follower 82 reaches the lock pocket 54, the needle guard 18 is no longer able to move relative to the plunger 12 and handle 10. The needle 30 is recessed in the needle guard 18 as shown in Figure 18A and locked from re-use.

### Assembly of Injection Device 8

The injection device 8 can be assembled in two sub-processes, basically a pre-assembly process in which a subassembly of the injector device (namely needle guard 18, handle 10 and overcap 20) is assembled to a state ready to receive a pre-filled syringe 26 and plunger 12, and then a final assembly process in which the remaining portions of the injector (plunger 12 along with the pre-filled syringe 26 and spring 16) are assembled with the subassembly to form a completed injection device which is ready for shipment to end-users or distributors. In one variation, the spring 16 could be inserted in the injector device in the pre-assembly process, preferably using a support bushing as described later in Figures 18-21. However, to prevent the spring from becoming dislodged in shipment to the site of final assembly, it is preferred to add the spring in the final assembly process.

In this embodiment, the first pre-assembly process involves the sub-steps of 1) inserting a hollow tubular needle guard (needle guard 18) into a hollow tubular handle 10, preferably using the guide features described previously, and then 2) placing a tamper-evidence overcap 20 over the needle guard 18 to engage either the needle guard 18 or the handle 10 or both. When this subassembly is completed, the handle 10 has the open proximal end for insertion of the syringe 26 and spring 16 and the plunger 12. This pre-assembly process may make use of conventional mandrels as necessary to hold the parts during assembly and spread retaining tabs and like components so that the parts can be assembled.

In the final assembly process, the combination of needle guard 18, handle 10 and overcap 20 resulting from step 2) is then assembled with the remaining components to form a completed device. This second part of the process involves the following sub-steps:
3) place a compression spring 16 over a pre-filled syringe assembly (Figure 2) including the syringe 26 and needle 30. The compression spring is inserted over the distal end of the syringe assembly so that the upper coil of the spring rests against the underside of the syringe flange 27.
4) insert the compression spring 16 and pre-filled syringe assembly combination into the needle guard 18 (from proximal to distal direction) and lock the spring 16 and pre-filled syringe assembly 26 to the needle guard/handle/overcap subassembly (by means of the snaps 84 of Figure 8A locing the syringe assembly in place). Alternatively and equivalently, the subassembly of the needle guard/handle/overcap can be slid over the syringe 26 and spring 16 combination from the distal end of the syringe 26 to the proximal end of the syringe 26 until the snaps 84 engage with the syringe 26.
5) insert the plunger 12 into open end of the handle 10. The end 15 of the plunger 12 will come into contact with the stopper 28 of the syringe 26.
6) lock the plunger 12 to the handle 10, e.g., by means of the tab features 40 of the plunger 12 (Figure 5C) engaging with the ledges 60 (Figures 7B and 7C) of the handle 10.

In the preferred embodiment the syringe assembly (Figure 6) includes a needle shield 32A and 32B covering the tip of a needle 30, and wherein step 2) of the above process comprises the sub-steps of 2a) placing an inner tamper evidence overcap 22 over the needle guard 18, 2b) placing a needle shield-engaging device (retainer 24) over the distal end of the overcap/needle guard combination; and then 2c) placing an outer tamper evidence overcap 20 over the inner tamper evidence overcap 22 and needle shield-engaging retainer 24 and securing the inner and outer tamper evidence overcaps 20 and 22 to each other (e.g. by virtue of engagement of the resilient ledges 196 of the inner overcap 22 with the pocket feature 110 of the overcap 20). This action retains the retainer 24 securely between the inner and outer overcaps. The retainer 24 ideally has some limited amount of play or float both transversely and axially (e.g., approximately 1 mm) in the space between the inner and outer overcaps in order to facilitate reliable insertion of the needle sheath into the retainer 24 during assembly.

During step 4), when the syringe 26 and needle sheath 32 are inserted into the needle guard/overcap subassembly the needle sheath 32 is inserted into engagement with the needle shield-engaging retainer 24, that is, the fingers 124 of the retainer 24 (Figure 10G) frictionally engage the needle sheath as shown in Figures 2 and 3. In the illustrated embodiment, the needle sheath 32 is made of a resilient material such that the end of the sheath 32 can move past the fingers and when the overcap 20 is removed the fingers 124 grip the sheath 32 and remove it from the needle 30 as described previously.

### Additional Alternative Embodiments

Many variations can be made to the disclosed embodiments without departure from the scope of the invention. Several particularly noteworthy variations will be noted in this section.

### 1. Track follower on plunger

The embodiment of Figures 1-18 includes the track follower as a feature of the needle guard 18 and the track as a feature of the plunger 12, but these positions can be reversed. In other words, the plunger 12 could be constructed to have a resilient track follower which projects in an axial direction and which engages with a slot, groove or other type of track feature formed on the external surface of the needle guard 18 to provide the same functionality as described herein.

Furthermore, the design could be such that only one track and track follower is used, or, alternatively, two or more than two tracks and track followers are used.

### 2. Track on handle

As another variation, the track follower could be re-oriented to face radially outward instead of radially inward as shown in Figure 8E and 8F, and the track that the track follower engages could be formed on the interior surface of the handle 10 instead of the top portion of the plunger 12.

Furthermore, in this embodiment the design could be such that only one track and track follower is used, or, alternatively, two or more than two tracks and track followers are used.

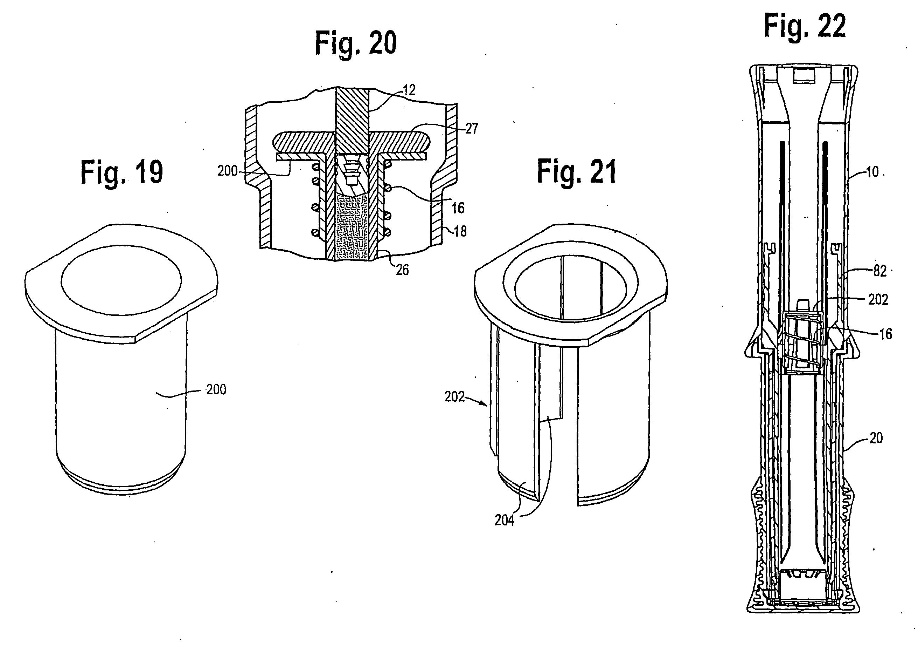

### 3. Optional spring support bushing (inner and outer) (Figures 19-22)

Figure 19 is a perspective view of a rigid, thin-walled spring support bushing 200 which may be included in the assembly. As shown in Figure 20, the bushing 200 is inserted over the syringe and rests within the internal diameter of the spring 16. The internal support bushing is captured underneath the needle guard snaps 84 (Figure 8A) and once assembled, under the syringe flange 27. The bushing rides freely with the syringe 26 under the flange 27 between the syringe body 26 and the longitudinal needle guard ribs above the shoulder 92 (Figure 8D and 8E). The internal bushing 200 is intended to provide support for the spring 16 during spring assembly into the needle guard 18 as well as assembly of the syringe 26 and spring combination into the rest of the injector device during assembly.

Figure 21 shows a variation of the spring support bushing concept in which the rigid, thin-walled support bushing 202 is positioned exterior to the spring 16 as shown in the cross-section of Figure 22. The bushing is slotted as indicated at 204 to provide relief for the needle guard ribs 90 during spring 16 compression. The external bushing 202 is also intended to provide location and support for the spring 16 during spring assembly into the needle guard 18 as well as assembly of the syringe 26 and spring combination into the rest of the injector device during assembly.

### Further explanations of the invention

The following characterizing clauses are further provided as additional description of preferred embodiments of the invention. The inclusion of reference numbers is the following clauses is to facilitate understanding of an exemplary embodiment but should not be considered limiting in this way.
1. A device for use in administering an injection to a human or animal subject, comprising, in combination:
   a syringe 26 comprising a body containing a medicament to be administered to the subject and having a needle 30 extending from the syringe body, the needle having a tip (Figure 6);
   a needle guard (needle guard 18, Figures 8A-8E) surrounding the syringe 26 and moveable relative to the syringe, the needle guard having at least one track follower 82 extending from the needle guard;
   a hollow tubular handle 10 having an interior region receiving the needle guard 18 and syringe 26;
   a plunger 12 coupled to the handle and positioned within the interior region of the handle 10, the plunger and handle moveable relative to the syringe body during administration of an injection, the plunger having a first end 15 engaging the syringe to expel medicament from the syringe upon movement of the plunger and handle relative to the syringe (see Figures 15-18);
   at least one track 42 for engagement with the track follower 82 of the needle guard 18; and
   a spring 16 acting on the syringe 26 and the needle guard 18;
   wherein the track follower 82 moves along the track 42 during movement of the handle 10 and plunger 12 relative to the needle guard 18 during the injection, and
   wherein the track further includes a lock position (54) wherein the spring 16 urges the needle guard relative to the handle 10 and plunger 12 such that the track follower 42 moves into the lock position 54 to lock the needle guard relative to the handle and plunger at the completion of the injection with the needle guard covering the tip of the needle to prevent an accidental needle stick following completion of the injection, as shown in Figures 15-18 and as described above.
2. The device of clause 1, wherein the plunger 12 includes a top portion 14 and wherein the track 42 is formed in the top portion of the plunger.
3. The device of clause 1 or clause 2, wherein the track is formed in the handle 10.
4. The device of any one of clauses 1-3, wherein the device further comprises a second track (see Figure 5C) and wherein the needle guard further comprises a second track follower 82 (Figure 8A), wherein the second track follower moves along the second track during movement of the handle and plunger relative to the needle guard during injection using the device, and wherein the second track further includes a lock position wherein the second track follower moves into the lock position to lock the needle guard relative to the handle and plunger at the completion of the injection.
5. The device of any of clauses 1-4, wherein the first track and the second track are formed in a portion of the plunger (see Figure 5C).
6. The device of any of clauses 1-5, wherein syringe includes a flange 27, and wherein the depth of penetration of the needle into the subject during the injection is controlled by contact between the syringe flange 27 and a portion of the needle guard (e.g., shoulder 93 in Figure 8D).
7. The device of any of clauses 1-6, wherein the device further comprises a removable tamper-evidence overcap 10 covering the needle guard 18, wherein the overcap comprises resilient means 194/198 preventing re-installation of the overcap onto the needle guard once the overcap has been removed from the needle guard.
8. The device of clause 7, wherein the syringe further comprises a needle sheath 32 covering the tip of the needle 30, and wherein the overcap further comprises a feature 24 engaging the needle sheath and removing the needle sheath from the needle simultaneous with the action of removing the overcap 10 from the needle guard 18.
9. The device of clause 7, wherein the syringe further comprises a flange 27 having an upper surface and a lower surface, and wherein the handle 10 further includes a feature (strut 63) for engaging the lower surface of the flange to limit axial movement of the syringe relative to the needle guard during the action of removing the overcap from the needle guard.
10. The device of claim any of clauses 1-9, wherein the track further comprises a feature (rib 50) wherein the track follower contacts the feature when the plunger is extended relative to the syringe body to substantially completely expel the medicament from the syringe, wherein the track follower is at least partially resilient, and wherein the construction of the track, the track feature and the track follower is such that the contact with the track follower with the track feature produces an audible sound indicating to the user of the device that the expelling of the medicament from the syringe is substantially completed.
11. The device of any of clauses 1-10, wherein the handle 10 and needle guard 18 comprise mutually engaging alignment features (ribs 64 on handle 10 and walls 88 on needle guard 18) for aligning the handle relative to the needle guard such that the needle guard is positioned within the handle in a manner such that the track follower 82 is aligned with the track 42.
12. The device of any of clause 1-11, wherein the needle guard is made from a transparent material.
13. The device of any of clauses 1-12, wherein the spring 16 comprises a helical coil spring and wherein the device further comprises a bushing 200 placed within the helical coil spring (Figure 20).
14. The device of any of clauses 1-12, wherein the spring comprises a helical coil spring and wherein the device further comprises a bushing 202 surrounding the helical coil spring (Figure 22).
15. An injection assembly for use with an injection device, comprising, in combination:
   a syringe 26 comprising a body containing a medicament to be administered to the subject and having a stopper 28 within the body and a needle 30 extending from the body; and
   a plunger 12 coupled the syringe, the plunger having a first end 15 engaging the stopper to expel medicament from the needle upon movement of the plunger 12 relative to the syringe body;
   wherein the plunger further comprises at least one track 42 for engagement with a track follower 82 of the injection device, the track having a lock position 54.
16. The assembly of clause 15, wherein the syringe further comprises a flange 27 and wherein the assembly further comprises a spring 16 positioned over the syringe and seating against the flange 27 of the syringe 26.
17. The assembly of clause 15 or clause 16, wherein the track 40 further includes a feature 50 wherein the track follower contacts the feature when the plunger is extended relative to the syringe body to substantially completely expel the medicament from the syringe, wherein the track follower is at least partially resilient, and wherein the construction of the track, the track feature and the track follower is such that the contact with the track follower with the track feature 50 produces an audible sound indicating to the user of the device that the expelling of the medicament from the syringe is substantially completed.
18. An injection assembly for use with an injection device, comprising, in combination:
   a syringe 26 comprising a body containing a medicament to be administered to the subject and having a stopper 28 within the body and a needle 30 extending from the body; and
   a plunger 12 coupled to the syringe, the plunger 12 having a first end 15 engaging the stopper 28 to expel medicament from the needle upon movement of the plunger relative to the syringe body;
   wherein the plunger further comprises at least one track follower for engagement with a track incorporated into the injection device, the track having a lock position. As explained above the arrangement of the track and track follower can be interchanged from the configuration shown in the drawings, such as for example with the plunger having a track follower and the track being formed on the needle guard.
19. The injection assembly of clause 18, wherein the syringe further comprises a flange 27 and wherein the assembly further comprises a spring 16 positioned over the syringe and seating against the flange of the syringe.
20. The injection assembly of clause 18 or clause 19, wherein the track follower 82 comprises an elongate flexible feature.
21. An injection device for receiving a syringe and plunger combination, the syringe containing a medicament for administration to a human or animal subject, the syringe further having a needle 30 and a needle sheath 32, the syringe further coupled to a plunger, the assembly comprising, in combination:
   a hollow tubular handle 10 having an open first end;
   a needle guard 18 positioned concentrically within the handle 10 having at least one track follower 82 extending from the needle guard and features (e.g., snaps 84 or the equivalent) for engagement with the syringe 26;
   a removable tamper-evidence overcap assembly 20, wherein the overcap assembly comprises resilient means (finger 194 and ledge 198 or equivalent) for engaging the needle guard and preventing re-installation of the overcap 20 onto the needle guard 18 once the overcap 20 has been removed from the needle guard 18; and
   a feature within the overcap assembly for engaging the needle sheath 32 the feature adapted to remove the needle sheath from the needle 30 simultaneous with the action of removing the overcap assembly 20 from the needle guard 18. The feature can be molded into the overcap or may be a separate part or piece, such as the retainer 24 of Figure 2.
22. The injection device of clause 21, wherein the syringe further comprises a flange 27 having an upper surface and a lower surface, and wherein the handle 10 further includes a feature 63 for engaging the lower surface of the flange 27 to limit axial movement of the syringe relative to the needle guard during the action of removing the overcap assembly from the needle guard.
23. The injection device of any of clauses 21-22, wherein the plunger 12 is constructed to engage with the handle 10 and close the open first end of the handle 10.
24. The injection device of any of clauses 21-23, wherein the feature within the overcap assembly comprises a retainer 24 and wherein the retainer makes a friction fit with the needle sheath.
25. The device of claim any of clauses 21-24, wherein needle guard 18 is made from a clear material.
26. In an injection device for injection of a medicament from a syringe into a human or animal subject, the improvement comprising, in combination:
   at least one track and at least one track follower, the track and track follower incorporated into an injection device supporting the syringe and facilitating injection of the medicament from the syringe,
   wherein the track further comprises a feature (such as rib or other feature) wherein the track follower contacts the feature when the medicament is substantially completely expelled from the syringe during the injection, wherein the track follower is at least partially resilient, and wherein the construction of the track, the feature and the track follower is such that the contact with the track follower with the feature produces an audible sound indicating to the user of the device that the injection of the medicament from the syringe is substantially completed.
27. The improvement of clause 26, wherein the injection device further comprises a plunger engaging with the syringe to expel the medicament from the syringe and a needle guard surrounding the syringe, and wherein the track 42 is incorporated into a plunger 12 and wherein the track follower comprises an elongate flexible feature 82 extending from the needle guard.
28. The improvement of clause 26, the injection device further comprises a plunger engaging with the syringe to expel the medicament from the syringe and a needle guard surrounding the syringe, and wherein the improvement comprises first and second track followers 82 and first and second tracks 42, the tracks are incorporated into the plunger 12 and wherein the track followers 82 comprise elongate flexible features extending from the needle guard.
29. The improvement of clause 26, wherein the track is constructed with a locking feature such that the track follower makes an audible sound when the track follower enters the locking feature.
30. An apparatus for injection of a medicament from a syringe having a needle with a tip into a human or animal subject, comprising, in combination:
   an injection device supporting the syringe and facilitating injection of the medicament from the syringe, the injection device including a needle guard 18;
   at least one track and at least one track follower;
   a spring acting on the syringe and the injection device;
   wherein the track follower moves along the track during an injection using the device; and
   wherein the track further includes a lock position wherein the spring urges the needle guard such that the track follower moves into the lock position to lock the needle guard relative to the syringe at the completion of the injection with the needle guard covering the tip of the needle to prevent an accidental needle stick following completion of the injection.
31. The apparatus of clause 30, wherein the track is incorporated into a plunger engaging with the syringe to expel a medicament from the syringe and wherein the track follower comprises an elongate flexible feature extending from the needle guard.
32. The apparatus of clause 30, wherein the apparatus comprises first and second track followers and first and second tracks, the tracks incorporated into a plunger engaging with the syringe to expel medicament from the syringe and wherein the track followers comprise elongate flexible features extending from the needle guard.
33. A method of assembly of an injection device, comprising the steps of:
   1) inserting a hollow tubular needle guard into a hollow tubular handle;
   2) placing a tamper-evidence overcap over the needle guard;
   3) placing a compression spring over a pre-filled syringe assembly;
   4) inserting the compression spring and pre-filled syringe assembly combination into the needle guard and locking the spring and pre-filled syringe assembly to the needle guard;
   5) inserting a plunger having a first end into the handle such that the first end of the plunger engages with a body of the syringe assembly; and
   6) locking the plunger to the handle.
34. The method of clause 33, wherein the syringe assembly includes a needle sheath 32 covering a tip of the syringe needle, and wherein step 2) comprises the sub-steps of:
   2a) placing an inner tamper evidence overcap 22 over the needle guard 18;
   2b) placing a needle sheath-engaging device 24 over the inner overcap 22 and needle guard 18 combination; and
   2c) placing an outer tamper evidence overcap 20 over the inner tamper evidence overcap 20 and needle sheath-engaging device 24 and securing the inner and outer tamper evidence overcaps to each other.
35. The method of clause 33 or 34, and wherein during step 4) the needle sheath is inserted into engagement with the needle sheath -engaging device.
36. The method of clause 34, wherein the needle shield-engaging device comprises a retainer having a plurality of fingers 124 and wherein the needle sheath is made from a resilient material whereby that the fingers frictionally grip the needle sheath at the completion of step 4).
37. The method of any of clauses 33-34, further comprising the step of inserting a spring support bushing over the syringe.
38. The method of clause 37, wherein the spring support bushing is positioned inside the compression spring.
39. The method of clause 37, wherein the spring support bushing is positioned exterior of the compression spring.
40. The device of clause 1, wherein the plunger is constructed with an axial length so as to engage a stopper in the syringe, wherein the syringe is only partially filled with a medicament.
41. The device of clause 40, wherein the handle has an open first end, the plunger has a top portion which is coupled to the handle and closes off the open first end, and wherein the track is formed in the top portion of the handle.
42. The device of clause 41, wherein the plunger has a proximal end and wherein the track is spaced from the proximal end of the plunger.
43. The device of clause 1, wherein the plunger has a proximal end and a distal end, and wherein the distal end further comprises a flared feature engaging the syringe and resisting movement of the syringe relative to the plunger in the proximal direction.
44. In an injection device having a syringe having a needle sheath and a needle guard, the improvement comprising:
   providing a tamper-resistant overcap covering the syringe and needle guard prior to use of the injection device, wherein the overcap is removed from the needle guard by applying an axial force to the overcap, and wherein the overcap is constructed with a feature which (1) pulls the needle sheath off the needle simultaneous with the removal of the overcap from the needle guard and (2) thereafter securely retains the needle sheath within the overcap.
45. The improvement of clause 44, wherein the injection device includes a hollow tubular handle which is grasped to administer an injection, and wherein the overcap fits into engagement with the handle.
46. The improvement of clause 45, wherein both the handle and the overcap comprise one or more flat surfaces to inhibit rolling of the injection device when placed on a hard flat horizontal surface.
47. The improvement of clause 44, wherein the overcap comprises an inner overcap and an outer overcap and wherein the feature comprises a retainer positioned between the inner and outer overcaps.
48. The improvement of clause 47, wherein the retainer comprises a hat-shaped member with a plurality of fingers for gripping the needle sheath.
49. The improvement of clause 50, wherein the retainer floats between the inner and outer overcaps.
50. In an injection device comprising a syringe having a tubular body for containing a medicament, a stopper moveable within the tubular body, a needle, a needle sheath covering the needle, and a plunger acting on the stopper to expel medicament from the syringe, the improvement comprising:
   providing a feature in the injection device limiting the axial movement of the syringe relative to the plunger when the needle sheath is removed from the needle.
51. The improvement of clause 50, wherein the plunger comprises a distal end engaging the stopper and wherein the feature comprises a feature formed in the distal end of the plunger which engages the tubular body of the syringe.
52. The improvement of clause 51, wherein the feature comprise a flared feature.
53. The improvement of clause 51, wherein the injection device further comprises a hollow tubular handle coupled to the plunger, and wherein the feature comprises a strut projecting from the handle and engaging the syringe to limit the axial movement of the syringe.

While a number of exemplary aspects and embodiments have been discussed above, those of skill in the art will recognize that still further modifications, permutations, additions and sub-combinations thereof of the features of the disclosed embodiments are still possible. It is therefore intended that the following appended claims and claims hereafter introduced are interpreted to include all such modifications, permutations, additions and sub-combinations as are within their true scope.

## Claims

1. A device for injection of a medicament from a syringe having a needle with a tip into a human or animal subject, comprising, in combination:
an injection device supporting the syringe and facilitating injection of the medicament from the syringe, the injection device including a needle guard;
at least one track and at least one track follower;
a spring acting on the syringe and the injection device;
wherein the track follower moves along the track during an injection using the device; and
wherein the track further includes a lock position wherein the spring urges the needle guard such that the track follower moves into the lock position to lock the needle guard relative to the syringe at the completion of the injection with the needle guard covering the tip of the needle to prevent an accidental needle stick following completion of the injection.

2. A device as claimed in claim 1, wherein the track is incorporated into a plunger engaging with the syringe to expel a medicament from the syringe and wherein the track follower comprises an elongate feature extending from the needle guard.

3. A device as claimed in claim 1, wherein the syringe comprises a body containing the medicament to be administered to the subject and the needle extends from the syringe body; and the needle guard surrounds the syringe and is moveable relative to the syringe, the needle guard having the at least one trade follower extending from the needle guard; and the apparatus further comprises a hollow tubular handle having an interior region receiving the needle guard and syringe; a plunger coupled to the handle and positioned within the interior region of the handle, the plunger and handle moveable relative to the syringe body during administration of an injection, the plunger having a first end engaging the syringe to expel medicament from the syringe upon movement of the plunger and handle relative to the syringe.

4. A device for use in administering an injection to a human or animal subject, comprising, in combination:
a syringe comprising a body containing a medicament to be administered to the subject and having a needle extending from the syringe body, the needle having a tip;
a needle guard surrounding the syringe and moveable relative to the syringe, the needle guard having at least one track follower extending from the needle guard;
a hollow tubular handle having an interior region receiving the needle guard and syringe;
a plunger coupled to the handle and positioned within the interior region of the handle, the plunger and handle moveable relative to the syringe body during administration of an injection, the plunger having a first end engaging the syringe to expel medicament from the syringe upon movement of the plunger and handle relative to the syringe;
at least one track for engagement with the track follower of the needle guard; and
a spring acting on the syringe and the needle guard;
wherein the track follower moves along the track during movement of the handle and plunger relative to the needle guard during the injection, and
wherein the track further includes a lock position wherein the spring urges the needle guard relative to the handle and plunger such that the track follower moves into the lock position to lock the needle guard relative to the handle and plunger at the completion of the injection with the needle guard covering the tip of the needle to prevent an accidental needle stick following completion of the injection.

5. A device as claimed in claim 3 or 4, wherein the plunger includes a top portion and wherein the track is formed in the top portion of the plunger.

6. A device as claimed in claim 3 or 4, wherein the track is formed in the handle.

7. A device as claimed in claim 3 or 4, wherein the device further comprises a second track and wherein the needle guard further comprises a second track follower,
wherein the second track follower moves along the second track during movement of the handle and plunger relative to the needle guard during injection using the device, and
wherein the second track further includes a lock position wherein the second track follower moves into the lock position to lock the needle guard relative to the handle and plunger at the completion of the injection.

8. A device as claimed in claim 7, wherein the first track and the second track are formed in a portion of the plunger.

9. A device as claimed in any of claims 3 to 8, wherein the syringe includes a flange, and wherein the depth of penetration of the needle into the subject during the injection is controlled by contact between the syringe flange and a portion of the needle guard.

10. A device as claimed in any of claims 3 to 9, wherein the device further comprises a removable tamper-evidence overcap covering the needle guard, wherein the overcap comprises resilient means for engaging the needle guard and inhibiting re-installation of the overcap onto the needle guard once the overcap has been removed from the needle guard.

11. A device as claimed in claim 10, wherein the syringe further comprises a needle sheath covering the tip of the needle, and wherein the overcap further comprises a feature engaging the needle sheath and removing the needle sheath from the needle simultaneous with the action of removing the overcap from the needle guard.

12. A device as claimed in claim 10 or 11, wherein the syringe further comprises a flange having an upper surface and a lower surface, and wherein the handle further includes a feature for engaging the lower surface of the flange to limit axial movement of the syringe relative to the needle guard during the action of removing the overcap from the needle guard.

13. A device as claimed in any of claims 3 to 12, wherein the track further comprises a feature wherein the track follower contacts the feature when the plunger is extended relative to the syringe body to substantially completely expel the medicament from the syringe, wherein the track follower is at least partially resilient, and wherein the construction of the track, the track feature and the track follower is such that the contact with the track follower with the track feature produces an audible sound indicating to the user of the device that the expelling of the medicament from the syringe is substantially completed.

14. A device as claimed in any of claims 3 to 13, wherein the handle and needle guard comprise mutually engaging features for aligning the handle relative to the needle guard such that the needle guard is positioned within the handle in a manner such that the track follower is aligned with the track.

15. A device as claimed in any of claims 3 to 14, wherein the needle guard is made from a transparent material.

16. A device as claimed in any of claims 3 to 15, wherein the spring comprises a helical coil spring and wherein the device further comprises a bushing placed within the helical coil spring.

17. A device as claimed in any of claims 3 to 15, wherein the spring comprises a helical coil spring and wherein the device further comprises a bushing surrounding the helical coil spring.

18. An injection assembly for use with an injection device, comprising, in combination:
a syringe comprising a body containing a medicament to be administered to a subject and having a stopper within the body and a needle extending from the body; and
a plunger having a first end engaging the stopper to expel medicament from the needle upon movement of the plunger relative to the syringe body;
wherein the plunger further comprises at least one track for engagement with a track follower of the injection device, the track having a lock position.

19. An assembly as claimed in claim 18, wherein the syringe further comprises a flange and wherein the assembly further comprises a spring positioned over the syringe and seating against the flange of the syringe.

20. An assembly as claimed in claim 18 or 19, wherein the track further includes a feature wherein the track follower contacts the feature when the plunger is extended relative to the syringe body to substantially completely expel the medicament from the syringe, wherein the track follower is at least partially resilient, and wherein the construction of the track, the track feature and the track follower is such that the contact with the track follower with the track feature produces an audible sound indicating to the user of the device that the expelling of the medicament from the syringe is substantially completed.

21. An injection assembly for use with an injection device, comprising, in combination:
a syringe comprising a body containing a medicament to be administered to the subject and having a stopper within the body and a needle extending from the body; and
a plunger having a first end engaging the stopper to expel medicament from the needle upon movement of the plunger relative to the syringe body;
wherein the plunger further comprises at least one track follower for engagement with a track incorporated into the injection device, the track having a lock position.

22. An injection assembly as claimed in claim 21, wherein the syringe further comprises a flange and wherein the assembly further comprises a spring positioned over the syringe and seating against the flange of the syringe.

23. An injection assembly as claimed in claim 21 or 22, wherein the track follower comprises an elongate flexible feature.

24. An injection device for receiving a syringe and plunger combination, the syringe containing a medicament for administration to a human or animal subject, the syringe further having a needle and a needle sheath, the syringe further coupled to a plunger, comprising, in combination:
a hollow tubular handle having an open first end;
a needle guard positioned within the handle having at least one track follower extending from the needle guard and having features for engagement with the syringe;
a removable tamper-evidence overcap assembly, wherein the overcap assembly comprises means for engaging the needle guard and inhibiting re-installation of the overcap onto the needle guard once the overcap has been removed from the needle guard; and
a feature within the overcap assembly for engaging the needle sheath, the feature adapted to remove the needle sheath from the needle simultaneous with the action of removing the overcap assembly from the needle guard.

25. An injection device as claimed in claim 24, wherein the syringe further comprises a flange having an upper surface and a lower surface, and wherein the handle further includes a feature for engaging the lower surface of the flange to limit axial movement of the syringe relative to the needle guard during the action of removing the overcap assembly from the needle guard.

26. An injection device as claimed in claim 24 or 25, wherein the plunger is constructed to engage with the handle and close the open first end of the handle.

27. An injection device as claimed in claim 24, 25 or 26, wherein the feature within the overcap assembly comprises a retainer and wherein the retainer makes a friction fit with the needle sheath.

28. An injection device as claimed in any of claims 24 to 27, wherein needle guard is made from a transparent material.

29. An injection device for injection of a medicament from a syringe into a human or animal subject, comprising, in combination:
at least one track and at least one track follower,
wherein the track further comprises a feature wherein the track follower contacts the feature when the medicament is substantially completely expelled from the syringe during the injection, wherein the track follower is at least partially resilient, and wherein the construction of the track, the feature and the track follower is such that the contact with the track follower with the feature produces an audible sound indicating to the user of the device that the injection of the medicament from the syringe is substantially completed.

30. a device as claimed in claim 29, wherein the injection device further comprises a plunger engaging with the syringe to expel the medicament from the syringe and a needle guard surrounding the syringe, and wherein the track is incorporated into the plunger, and wherein the track follower comprises an elongate feature extending from the needle guard.

31. A device as claimed in claim 29, wherein the injection device further comprises a plunger engaging with the syringe to expel the medicament from the syringe and a needle guard surrounding the syringe, and first and second track followers and first and second tracks, the tracks are incorporated into the plunger and wherein the track followers comprise elongate features extending from the needle guard.

32. A device as claimed in claim 29 to 30, wherein the track is constructed with a locking feature such that the track follower makes an audible sound when the track follower enters the locking feature.

33. A device as claimed in claim 1 or 2, wherein the apparatus comprises first and second track followers and first and second tracks, the tracks incorporated into a plunger engaging with the syringe to expel medicament from the syringe and wherein the track followers comprise elongate features extending from the needle guard.

34. A method of assembly of an injection device, comprising the steps of:
1) inserting a hollow tubular needle guard into a hollow tubular handle;
2) placing a tamper-evidence overcap over the needle guard;
3) placing a compression spring over a pre-filled syringe assembly;
4) inserting the compression spring and pre-filled syringe assembly combination into the needle guard and locking the spring and pre-filled syringe assembly to the needle guard;
5) inserting a plunger having a first end into the handle such that the first end of the plunger engages with the syringe assembly; and
6) locking the plunger to the handle.

35. A method as claimed in claim 34, wherein the syringe assembly includes a needle sheath covering a tip of a needle, and wherein step 2) comprises the sub-steps of:
2a) placing an inner tamper evidence overcap over the needle guard;
2b) placing a needle shield-engaging device onto the inner tamper evidence overcap and needle guard; and
2c) placing an outer tamper evidence overcap over the inner tamper evidence overcap and needle shield-engaging device and securing the inner and outer tamper evidence overcaps to each other.

36. A method as claimed in claim 35, and wherein during step 4) the needle sheath is inserted into engagement with the needle sheath-engaging device.

37. A method as claimed in claim 35, wherein the needle sheath-engaging device comprises a retainer having a plurality of fingers and wherein the fingers frictionally grip the needle sheath at the completion of step 4).

38. A method as claimed in any of claims 34 to 37, further comprising the step of inserting a spring support bushing over the syringe.

39. A method as claimed in claim 38, wherein the spring support bushing is positioned inside the compression spring.

40. A method as claimed in claim 38, wherein the spring support bushing is positioned exterior of the compression spring.

41. A device as claimed in any of claims 3 to 17, wherein the plunger is constructed with an axial length so as to engage a stopper in the syringe, wherein the syringe is only partially filled with a medicament.

42. A device as claimed in claim 41, wherein the handle has an open first end, the plunger has a top portion which is coupled to the handle and closes off the open first end, and wherein the track is formed in the top portion of the handle.

43. A device as claimed in claim 42, wherein the plunger has a proximal end and wherein the track is spaced from the proximal end of the plunger.

44. A device as claimed in any of claims 3 to 17 or 41 to 43, wherein the plunger has a proximal end and a distal end, and wherein the distal end further comprises a flared feature engaging the syringe and resisting movement of the syringe relative to the plunger in the proximal direction.

45. An injection device having a syringe having a needle sheath and a needle guard, comprising:
a tamper-resistant overcap covering the syringe and needle guard prior to use of the injection device, wherein the overcap is removed from the needle guard by applying an axial force to the overcap, and wherein the overcap is constructed with a feature which (1) pulls the needle sheath off the needle simultaneous with the removal of the overcap from the needle guard and (2) thereafter securely retains the needle sheath within the overcap.

46. A device as claimed in claim 45, wherein the injection device includes a hollow tubular handle which is grasped to administer an injection, and wherein the overcap fits into engagement with the handle.

47. A device as claimed in claim 46, wherein both the handle and the overcap comprise one or more flat surfaces to inhibit rolling of the injection device when placed on a hard flat horizontal surface.

48. A device as claimed in any of claims 45 to 47, wherein the overcap comprises an inner overcap and an outer overcap and wherein the feature comprises a retainer positioned between the inner and outer overcaps.

49. A device as claimed in claim 48, wherein the retainer comprises a hat-shaped member with a plurality of fingers for gripping the needle sheath.

50. A device as claimed in claim 48, wherein the retainer floats between the inner and outer overcaps.

51. An injection device comprising a syringe having a tubular body for containing a medicament, a stopper moveable within the tubular body, a needle, a needle sheath covering the needle, and a plunger acting on the stopper to expel medicament from the syringe, and a feature in the injection device limiting the axial movement of the syringe relative to the plunger when the needle sheath is removed from the needle.

52. A device as claimed in claim 51, wherein the plunger comprises a distal end engaging the stopper and wherein the feature comprises a feature formed in the distal end of the plunger which engages the tubular body of the syringe.

53. A device as claimed in claim 52, wherein the feature comprise a flared feature.

54. A device as claimed in claim 52, further comprising a hollow
tubular handle coupled to the plunger, and wherein the feature comprises a strut projecting from the handle and engaging the syringe to limit the axial movement of the syringe.
